(19) Europäisches Patentamt / European Patent Office / Office européen des brevets

(11) **EP 3 821 884 A1**

(12) **EUROPEAN PATENT APPLICATION**
published in accordance with Art. 153(4) EPC

(43) Date of publication:
**19.05.2021 Bulletin 2021/20**

(21) Application number: **19834376.6**

(22) Date of filing: **26.04.2019**

(51) Int Cl.:
***A61K 31/04** (2006.01)* ***A61K 31/06** (2006.01)*
***A61K 31/664** (2006.01)* ***C07C 205/38** (2006.01)*
***C07F 9/564** (2006.01)* ***C07B 55/00** (2006.01)*
***C07F 9/22** (2006.01)* ***A61P 29/00** (2006.01)*

(86) International application number:
**PCT/CN2019/084604**

(87) International publication number:
**WO 2020/010900 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **09.07.2018 CN 201810745871**

(71) Applicant: **Ascentawits Pharmaceuticals, Ltd.**
**Shenzhen, Guangdong 518118 (CN)**

(72) Inventors:
- **DUAN, Jianxin**
  **Shenzhen, Guangdong 518118 (CN)**
- **LI, Anrong**
  **Shenzhen, Guangdong 518118 (CN)**
- **MENG, Fanying**
  **San Francisco, California 94121 (US)**
- **JUNG, Donald T**
  **Cupertino, California 95014 (US)**

(74) Representative: **Nederlandsch Octrooibureau**
**P.O. Box 29720**
**2502 LS The Hague (NL)**

(54) **USE OF COMPOUND IN DRUG FOR PREVENTING, TREATING, OR ALLEVIATING PAIN**

(57) The present invention provides a use of a compound shown in Formula I/II in a drug for preventing, treating or alleviating pain

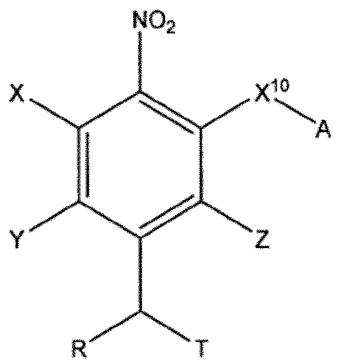

I

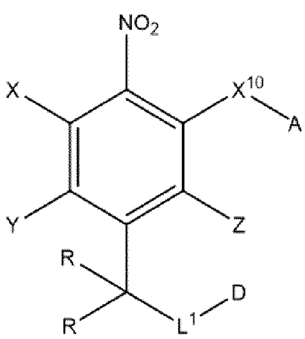

II

EP 3 821 884 A1

Fig. 1

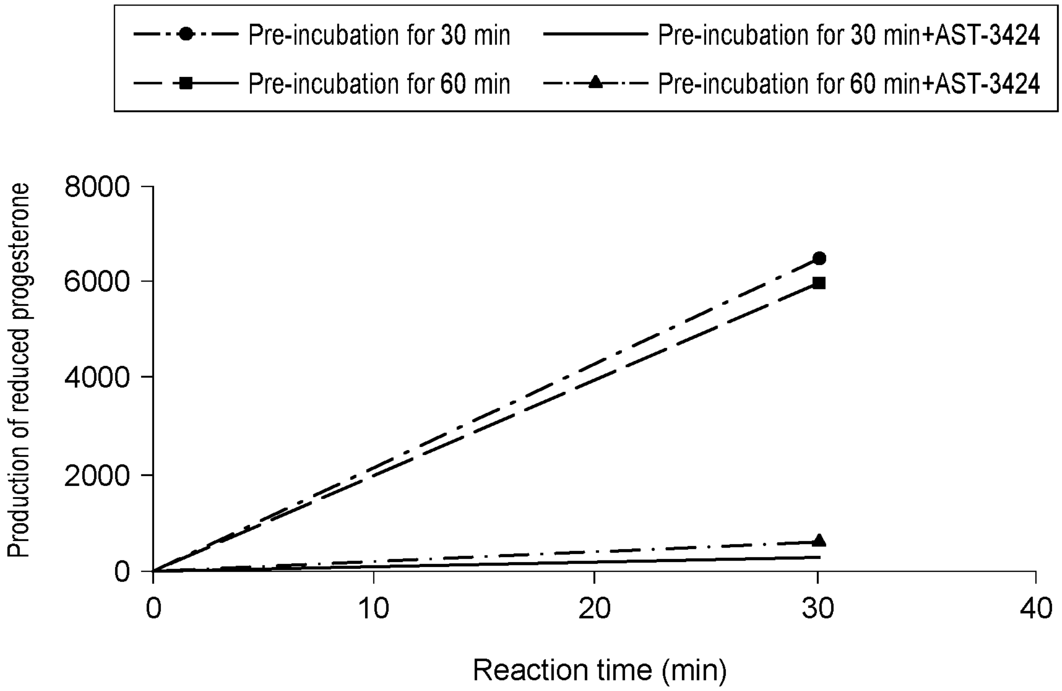
Pre-incubation for 30 min
Pre-incubation for 30 min+AST-3424
Pre-incubation for 60 min
Pre-incubation for 60 min+AST-3424
Production of reduced progesterone
8000
6000
4000
2000
0
0
10
20
30
40
Reaction time (min)

## Description

### Technical Field

The present invention relates to use of the compounds disclosed in Patent Application No. PCT/US2016/021581, Patent Application No. PCT/US2016/025665, and Patent Application No. PCT/US2016/062114 in the manufacture of a medicament for prevention, treatment or alleviation of pain.

### Background Art

Pain is a painful sensation caused by actual or potential tissue damage. It is both a protective response of the body and a common clinical symptom of many diseases.

The treatment of pain is very important in medicine. Traditional analgesic medicaments mainly include opioids and nonsteroidal anti-inflammatory drugs. Opioids are alkaloids extracted from opium (opium poppy) and *in vitro* and *in vivo* derivatives, which can relieve pain and produce a strong analgesic effect by interacting with specific central receptors. Nevertheless, chronic use of opioids can easily cause tolerance, dependence and addiction, and lead to such adverse reactions as respiratory inhibition and central nervous sedation. Currently, opioids are used for acute sharp pain and cancer intense pain. Nonsteroidal anti-inflammatory drugs are a class of anti-inflammatory drugs that do not contain steroid structures. They have, among others, anti-inflammatory, antirheumatic, analgesic, antipyretic and anticoagulant effects, and are widely used clinically for the relief of osteoarthritis, rheumatoid arthritis, various fevers and various pain symptoms. Nevertheless, such drugs only have moderate analgesic action and are suitable for mild and moderate chronic dull pain, but are not effective for sharp pain caused by direct stimulation of sensory endings. Additionally, they have adverse reactions such as gastrointestinal bleeding and cardiotoxicity.

Therefore, there remains a need in the art for developing new analgesic medicaments.

### Summary of the Invention

In order to solve the aforementioned problems, the present invention, based on the compounds or pharmaceutically acceptable salts, or solvates thereof as disclosed in Patent Application No. PCT/US2016/021581 (WO2016/145092), Patent Application No. PCT/US2016/025665 (WO2016/161342), and Patent Application No. PCT/US2016/062114 (WO2017/087428), provides use of those compounds or pharmaceutically acceptable salts, or solvates thereof in the manufacture of a medicament for prevention, treatment or alleviation of pain.

It has been proven that the compounds disclosed in the patent applications PCT/US2016/021581, PCT/US2016/025665 and PCT/US2016/062114, as internationally original, small molecule targeted therapeutic drugs with high tumor selectivity, have shown excellent anticancer effects in varoius preclinical cells and animal models. These compounds, as specific substrates of the aldo-keto reductase AKR1C3, can be quickly and effectively reduced only in cancer cells which overexpress AKR1C3, thereby releasing cytotoxins to result in highly selective cancer cell killing effects.

The research group contemplates that since the compounds disclosed in the aforementioned invention patent applications are used as specific substrates of the aldo-keto reductase AKR1C3 (hereinafter referred to as specific substrates), they are actually anticancer alkylating agent prodrugs, which are specifically activated under the action of the aldo-keto reductase AKR1C3 and are metabolized to produce a cytotoxic alkylating agent. Take AST-2870 (the compound TH-2870 disclosed in the aforementioned PCT/US2016/021581) as an example.

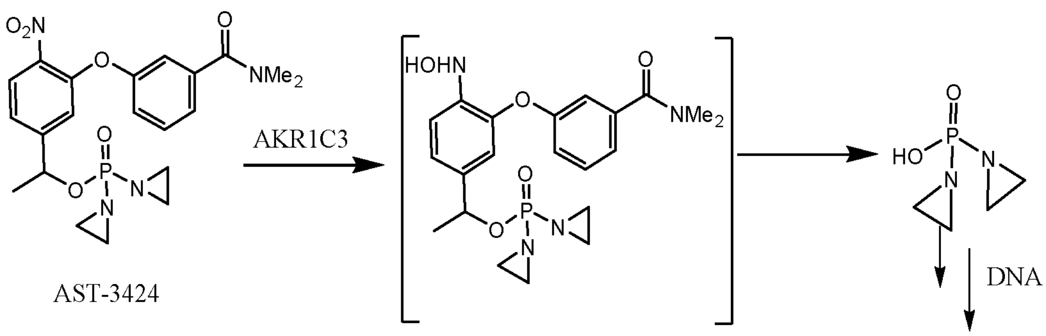


AST-3424

Stable Compound | Unstable intermediate | Cell death

Evidently, the aldo-keto reductase AKR1C3 needs to bind to the specific substrates.

According to the research literature (Samad T A, Moore K A, Sapirstein A, et al. Interleukin-1[beta]-mediated

induction of Cox-2 in the CNS contributes to inflammatory pain hypersensitivity[J]. Nature, 2001, 410(6827):471-5.; Baojian Z, Yanbing Y, Gele A, et al. Tanshinone IIA Attenuates Diabetic Peripheral Neuropathic Pain in Experimental Rats via Inhibiting Inflammation[J]. Evidence-Based Complementary and Alternative Medicine, 2018, 2018:1-8.; Lovering A, Ride J, Bunce C, et al. Crystal Structures of Prostaglandin D2 11-Ketoreductase (AKR1C3) in Complex with the Nonsteroidal Anti-Inflammatory Drugs Flufenamic Acid and Indomethacin[J]. Cancer Research, 2004, 64(5):1802-1810.; Matsuura K, Shiraishi H, Hara A, et al. Identification of a principal mRNA species for human 3alpha-hydroxysteroid dehydrogenase isoform (AKR1C3) that exhibits high prostaglandin D2 11-ketoreductase activity.[J]. Journal of Biochemistry, 1998, 124(5):940-6.), it is known that the aldo-keto reductase AKR1C3 plays an important catalytic role in the biochemical pathway of the conversion of prostaglandin H2/D2 to prostaglandin E2/F2:

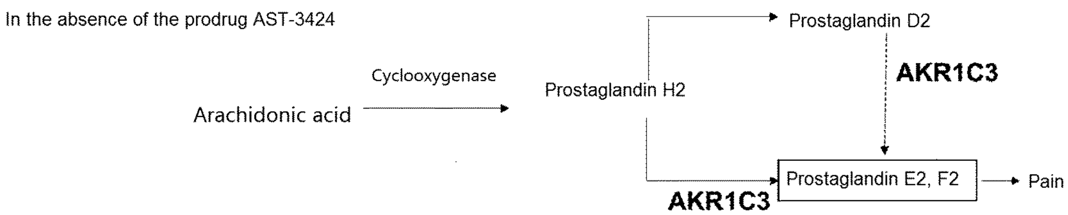


The inventors contemplate that since the specific substrates can bind to the aldo-keto reductase AKR1C3, can it be an inhibitor of the aldo-keto reductase AKR1C3, thereby reducing the level of prostaglandin E2/F2, and preventing, treating or alleviating the pain caused by cancer or inflammation?

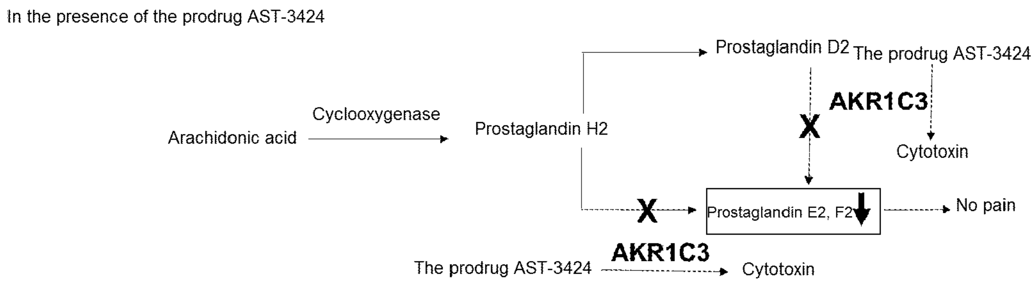


Regarding above assumptions, the inventors have designed experiments verifying that the aforementioned specific substrates, namely DNA alkylating agent targeting overexpressed aldo-keto reductase AKR1C3, can inhibit the activity of the aldo-keto reductase AKR1C3. Moreover, the animal experiment proves that the specific substrates can reduce the content of prostaglandin E2/F2 in the blood. Accordingly, it can be proved that the aforementioned specific substrates have an analgesic effect.

Regarding the analgesic effect of the aforementioned compounds, especially the use thereof in the manufacture of a medicament for preventing, treating or alleviating the pain caused by cancer or inflammation, the present invention provides the following technical solutions.

In one aspect, the present invention provides use of a compound of formula I or a pharmaceutically acceptable salt, or a solvate thereof in the manufacture of a medicament for preventing, treating or alleviating pain,

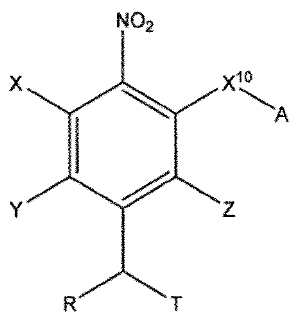

I

wherein

$X^{10}$ is O, S, SO, or $SO_2$;

A is $C_6$-$C_{10}$ aryl or substituted aryl, 5-15 membered heteroaryl or substituted heteroaryl, or $-N=CR^1R^2$, wherein each $R^1$ and $R^2$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;

each X, Y, and Z independently is hydrogen, CN, halo, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;

R is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;

each $R^{13}$ and $R^{14}$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl or ether, or $R^{13}$ and $R^{14}$ together with the nitrogen atom to which they are bonded to form 5-7 membered heterocyclyl group;

T comprises a phosphoramidate alkylating agent; and wherein the alkyl, alkenyl, alkynyl, cycloalkyl, aryl, heterocycle, heteroaryl, ether groups are optionally substituted.

Preferably, the compound is of formula I-A,

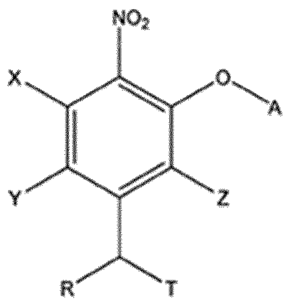

I-A

wherein the remaining variables are defined as above.

Alternatively, $X^{10}$ is S.

More preferably, in the compound, T is $OP(Z^1)(NR^{30}CH_2CH_2X^1)_2$, $OP(Z^1)(NR^{30}{}_2)(N(CH_2CH_2X^1)_2)$, $OP(Z^1)(N(CH_2)_2)_2$ or $OP(Z^1)(N(CH_2CH_2X^1)_2)_2$, wherein each $R^{30}$ independently is hydrogen or $C_1$-$C_6$ alkyl or two $R^{30}$ groups together with the nitrogen atom to which they are bonded to form 5-7 membered heterocyclyl group, $Z^1$ is O or S, and $X^1$ is Cl, Br, or OMs or other leaving groups.

Further preferably, in the compound, T is $OP(Z^1)(NHCH_2CH_2Cl)_2$, $OP(Z^1)(NHCH_2CH_2Br)_2$, $OP(Z^1)(NH_2)(N(CH_2CH_2X^1)_2)$, $OP(Z^1)(N(CH_2)_2)_2$ or $OP(Z^1)(N(CH_2CH_2Cl)_2)_2$, wherein $Z^1$ is O or S, and $X^1$ is Cl, Br, or OMs.

According to particular embodiments of the invention, in the compound, $Z^1$ is O or S.

According to particular embodiments of the invention, in the compound, T is $OP(O)(N(CH_2CH_2))_2$, $OP(O)(NHCH_2CH_2Cl)_2$, $OP(O)(NHCH_2CH_2Br)_2$ or $OP(O)(NH_2)(N(CH_2CH_2Cl)_2)$.

According to particular embodiments of the invention, in the compound, Z is hydrogen.

According to particular embodiments of the invention, in the compound, X is hydrogen.

According to particular embodiments of the invention, in the compound, Y is hydrogen or halo.

Preferably, in the compound, A is optionally substituted $C_6$-$C_{10}$ aryl.

More preferably, in the compound, A is optionally substituted phenyl.

Preferably, in the compound, A is optionally substituted 5-15 membered heteroaryl.

More preferably, in the compound, A is optionally substituted pyridyl.

More preferably, in the compound, A is $-N=CR^1R^2$ where $R^1$ and $R^2$ are defined as above.

According to particular embodiments of the invention, in the compound, R is hydrogen.

Preferably, in the compound, R is $C_1$-$C_6$ alkyl.

According to particular embodiments of the invention, in the compound, R is methyl. Regarding the use described herein, the compounds include individual diastereomers and other geometric isomers, and enantiomers, and mixtures

of enantiomers, diastereomers, and geometric isomers other than diastereomers.

The compound of formula I described above in the present patent is disclosed in PCT Application No. PCT/US2016/021581 (International Publication No. WO2016/145092), which corresponds to Chinese Patent Application No. CN201680015078.8 (Publication No. CN107530556A), the disclosure of which is incorporated herein by reference in its entirety. In this PCT application, the general structure of the compound, the radical substitution, the preferred general structure, the preferred specific compounds and the like are all described in detail, and the entire contents thereof are incorporated herein by reference. Therefore, the use of the compound as described above or the pharmaceutically acceptable salt, or the solvate thereof in the manufacture of a medicament for the prevention, treatment or alleviation of pain in the present invention covers all the contents about the compound or the pharmaceutically acceptable salt, or the solvate thereof as provided in PCT Application No. PCT/US2016/021581.

In another aspect, the invention provides use of a compound of formula II or a pharmaceutically acceptable salt, or a solvate thereof in the manufacture of a medicament for the prevention, treatment or alleviation of pain,

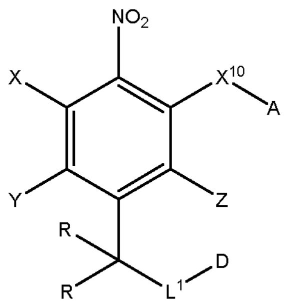

II

wherein

$X^{10}$ is O, S, SO, or $SO_2$;

A is $C_6$-$C_{10}$ aryl or substituted aryl, 5-15 membered heteroaryl or substituted heteroaryl, or $-N=CR^1R^2$, wherein each $R^1$ and $R^2$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;

each X, Y, and Z independently is hydrogen, CN, halo, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;

each R independently is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, - $CONR^{13}R^{14}$, or $-NR^{13}COR^{14}$;

each $R^{13}$ and $R^{14}$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl, or ether, or $R^{13}$ and $R^{14}$ together with the nitrogen atom to which they are bonded to form 5-7 membered heterocyclyl group;

$L^1$ and D are defined as follows:

$L^1$ is selected from:

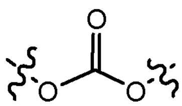, 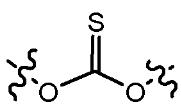, 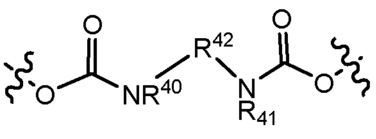, 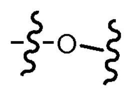,

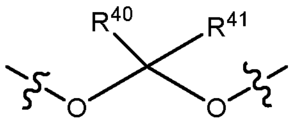 and 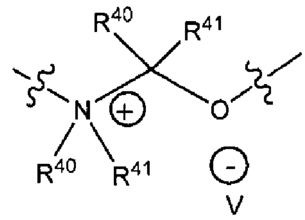 ;

wherein $R^{40}$ and $R^{41}$ are independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, or 5-15 membered heteroaryl; $R^{42}$ is $C_2$-$C_3$ alkylene or heteroalkylene optionally substituted with 1-3 $C_1$-$C_6$ alkyl, V(-) is any anion, preferably, a pharmaceutically acceptable anion, and

D is a moiety such that D-OH is an anticancer drug wherein OH is an aliphatic or a phenolic hydroxy group or is an OH moiety attached to a phosphorous atom as provided herein; in other words, D is the remaining group in the anticancer drug DOH after the hydroxyl group is removed therefrom;

alternatively

$L^1$ is:

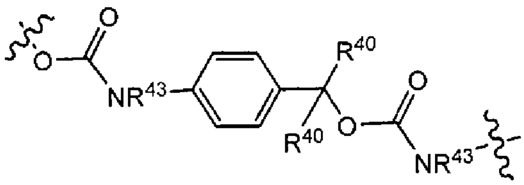 , 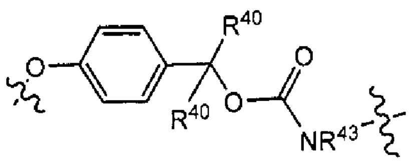

or

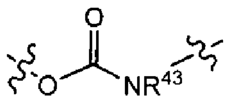 ;

wherein $R^{40}$ is defined as above, $R^{43}$ is hydrogen or together with D forms a heterocycle, and the phenyl moiety is optionally substituted, and

D is a moiety such that D-$NR^{43}$H is an anticancer drug; in other words, D is the remaining group in the anticancer drug D-$NR^{43}$H after amino or amine is removed therefrom;

alternatively

$L^1$ is a bond, -O-C($R^{40}R^{41}$)$_2$-, -O-C($R^{40}R^{41}$)-$NR^{40}R^{41}$(+)-C($R^{40}R^{41}$)- or

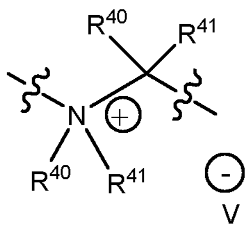 ,

wherein $R^{40}$, $R^{41}$ and V are defined as above, and

D is an anticancer drug containing a primary or a secondary amine, wherein the primary or the secondary amine is

bonded to $L^1$; and

wherein the alkyl, alkenyl, alkynyl, cycloalkyl, aryl, heterocycle, heteroaryl, and ether groups are optionally substituted.

Preferably, the compound is of formula II-A,

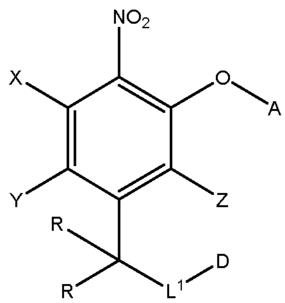
or
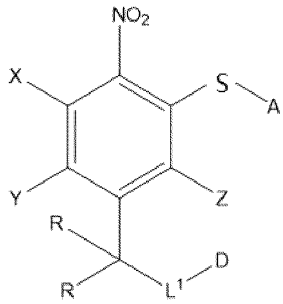

II-A

wherein the remaining variables are defined as above.

In another example, $X^{10}$ is S.

More preferably, the compound is of formula IIA-1:

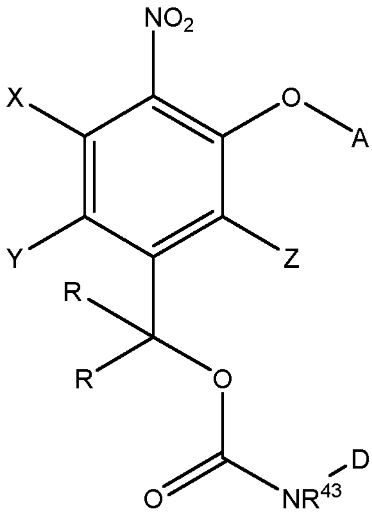
or
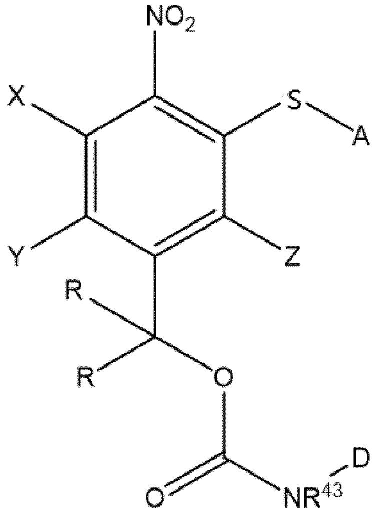

IIA-1

wherein the remaining variables are defined as above, and D is a moiety of the cytotoxic agent $HNR^{43}$-D containing a primary or a secondary amine group; in other words, D is the remaining group in the cytotoxic agent $HNR^{43}$-D after the primary or a secondary amine group is removed therefrom.

Alternatively, more preferably, the compound is of formula III-2 or IIIA-3:

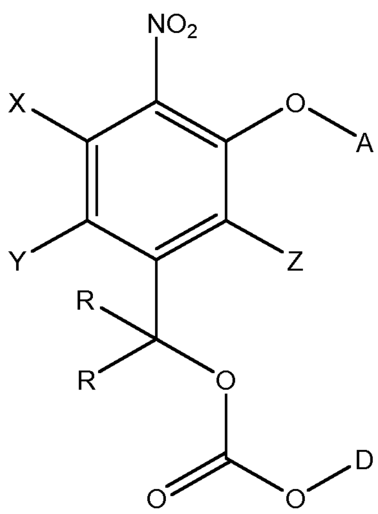

or

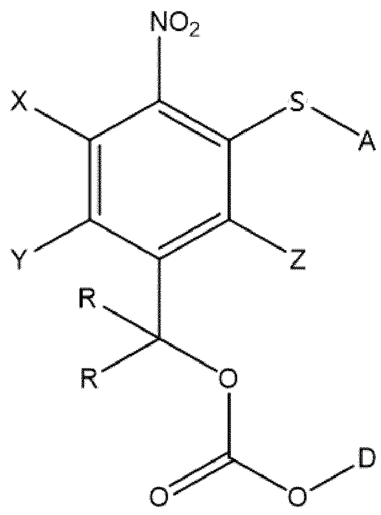

IIA-2

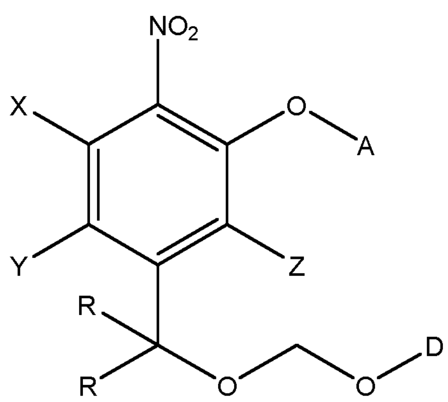

or

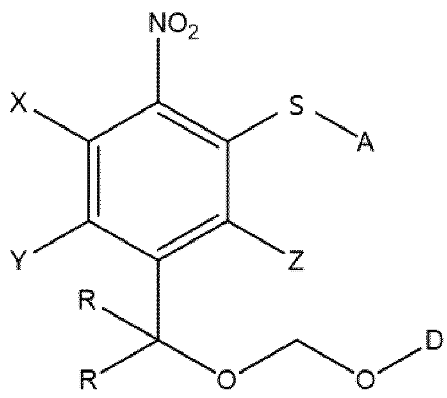

IIA-3

wherein the remaining variables are defined as above, and D is a moiety of the cytotoxic agent HO-D containing at least one hydroxyl group; in other words, D is the remaining group in the cytotoxic agent HO-D after the hydroxyl group is removed therefrom.

Alternatively, more preferably, the compound is of IIA-4, IIA-6 or IIA-6-i:

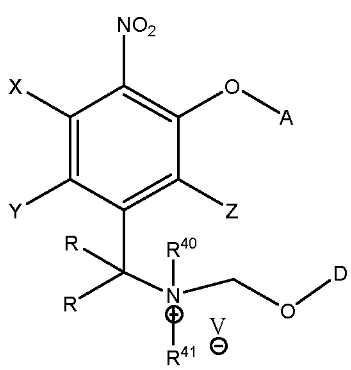

or

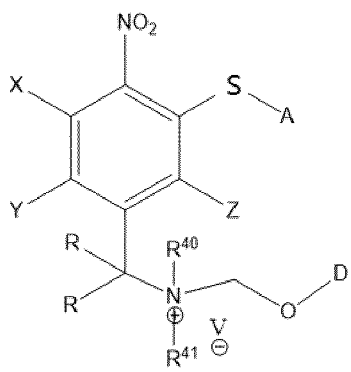

IIA-4

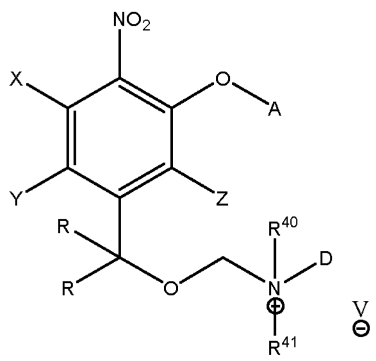

or

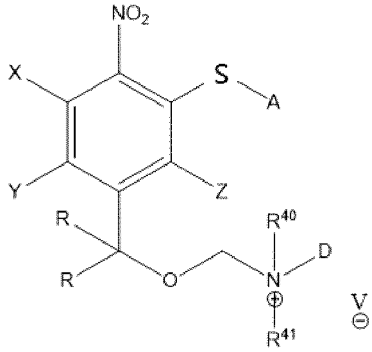

IIA-6-i

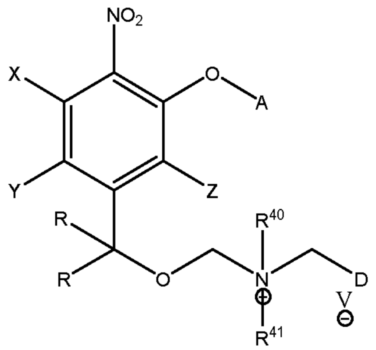

or

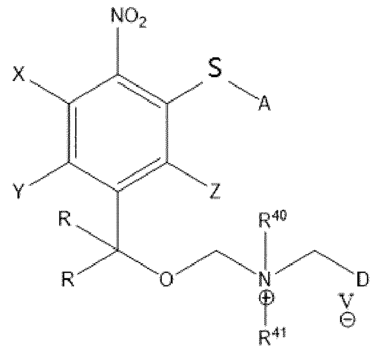

,

IIA-6

wherein the variables are defined as above; wherein in IIA-4, HO-D is cytotoxic agent containing at least one hydroxyl group; in other words, D is a moiety of the cytotoxic agent HO-D containing at least one hydroxyl group, or D is the remaining group in the cytotoxic agent HO-D after the hydroxyl group(s) is/are removed therefrom; in IIA-6-i, $DNR^{40}R^{41}$ is a drug; in other words, D is a moiety such that $DNR^{40}R^{41}$ is an anticancer drug or D is the remaining moiety in the anticancer $DNR^{40}R^{41}$ after $NR^{40}R^{41}$ is removed therefrom; and in IIA-6, D is a drug containing a secondary amine, wherein the secondary amine is bonded to the methylene group; in other words, D is a drug containing a secondary amine, and is bonded via the secondary amine contained therein to the methylene group so as to be linked to - $NR^{40}R^{41}$ as shown above.

Alternatively, more preferably, the compound is of IIA-5 or IIA-7:

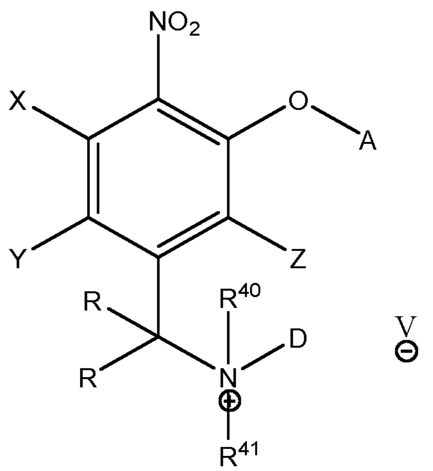

or

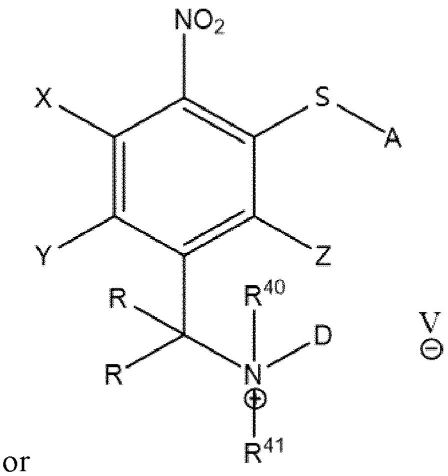

IIA-5

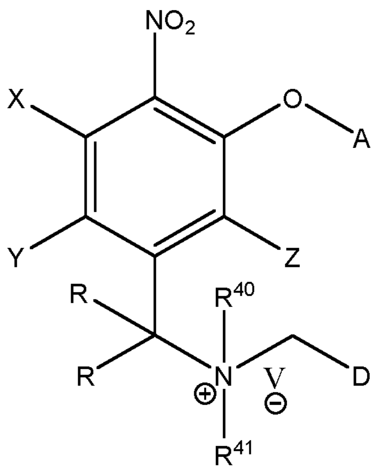

or

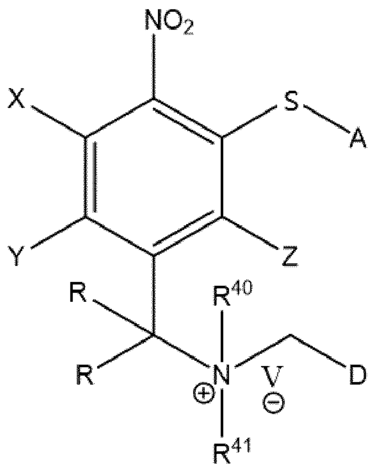

,

IIA-7

wherein the variables are defined as above; wherein in IIA-5, $DNR^{40}R^{41}$ is a drug; in other words, D is a moiety such that $DNR^{40}R^{41}$ is an anticancer drug or D is the remaining moiety in the anticancer $DNR^{40}R^{41}$ after $NR^{40}R^{41}$ is removed therefrom; and in IIA-7, D is a drug containing a secondary amine, wherein the secondary amine is bonded to the methylene group as shown above.

According to particular embodiments of the invention, in the compound, Z is hydrogen.

According to particular embodiments of the invention, in the compound, X is hydrogen.

According to particular embodiments of the invention, in the compound, Y is hydrogen or halo.

Preferably, in the compound, A is optionally substituted $C_6$-$C_{10}$ aryl.

More preferably, in the compound, A is optionally substituted phenyl.

Preferably, in the compound, A is optionally substituted 5-15 membered heteroaryl.

More preferably, in the compound, A is optionally substituted pyridyl.

More preferably, in the compound, A is $-N=CR^1R^2$, wherein $R^1$ and $R^2$ are defined as above.

According to particular embodiments of the invention, in the compound, each R is hydrogen.

Preferably, in the compound, one of the R groups is hydrogen and the other R group is $C_1$-$C_6$ alkyl, or both R groups are non-hydrogen substituents as defined above.

According to particular embodiments of the invention, in the compound, R is methyl.

Preferably, in the compound, each of $R^{40}$, $R^{41}$ and $R^{43}$ is independently hydrogen or methyl and $R^{42}$ is $-CH_2-CH_2-$ or $CH_2-C(Me)_2-$.

Particularly, as used herein, D excludes a phosphoramidate alkylating agent such as $-P(Z^1)(NR^{30}CH_2CH_2X^1)_2$, $-P(Z^1)(NR^{30}{}_2)(N(CH_2CH_2X^1)_2)$, $-P(Z^1)(N(CH_2CH_2))_2$ or - $P(Z^1)(N(CH_2CH_2X^1)_2)_2$, wherein each $R^{30}$ independently is hydrogen or $C_1$-$C_6$ alkyl or two $R^{30}$ groups together with the nitrogen atom to which they are bonded to form 5-7 membered heterocyclyl group, $Z^1$ is O or S, and $X^1$ is Cl, Br, or OMs or another leaving group.

Regarding the use described herein, the compounds include individual diastereomers and other geometric isomers, and enantiomers, and mixtures of enantiomers, diastereomers, and geometric isomers other than diastereomers.

The compound of formula II described above in the present patent is disclosed in PCT Application No. PCT/US2016/025665 (International Publication No. WO2016/161342), which corresponds to Chinese Patent Application No. CN201680020013.2 (Publication No. CN108136214A), the disclosure of which is incorporated herein by reference in its entirety. In this PCT application, the general structure of the compound, the radical substitution, the preferred general structure, the preferred specific compounds and the like are all described in detail, and the entire contents thereof are incorporated herein by reference. Therefore, the use of the compound as described above or the pharmaceutically acceptable salt, or the solvate thereof in the present invention in the manufacture of a medicament for the prevention, treatment or alleviation of pain covers all the contents about the compound or the pharmaceutically acceptable salt, or the solvate thereof as provided in PCT Application No. PCT/US2016/025665.

According to particular embodiments of the invention, the compound I is the compound AST-3424 as shown below:

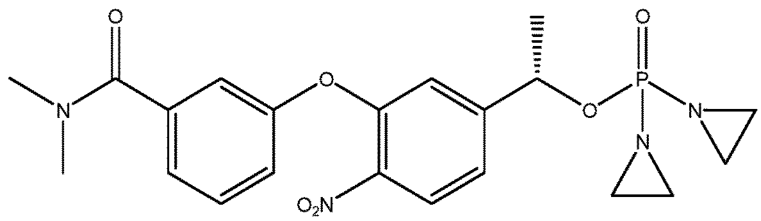

AST-3424 (The S-isomer of compound 2870 in the three aforementioned PCT applications)

The compounds for the use described herein may also be used in the form of their salts. In other words, the present invention provides use of pharmaceutically acceptable salts of the compounds as shown herein in the manufacture of a medicament for the prevention, treatment or alleviation of pain. Herein, the salts may be basic salts, including the salts of the compounds with an inorganic base (such as alkali metal hydroxide and alkaline earth metal hydroxide) or with an organic base (such as monoethanolamine, diethanolamine or triethanolamine). Alternatively, the salts may be acid salts, including the salts of the compounds with an inorganic acid (such as hydrochloric acid, hydrobromic acid, hydroiodic acid, nitric acid, perchloric acid, sulfuric acid or phosphoric acid) or with an organic acid (such as methanesulfonic acid, trifluoromethanesulfonic acid, ethanesulfonic acid, benzenesulfonic acid, p-toluenesulfonic acid, fumaric acid, oxalic acid, maleic acid and citric acid). It is a well-known technology in the art to select and prepare acceptable salts, solvates, and the like of a compound.

The compounds for the use described herein may also be used in the form of solvates. In other words, the present invention provides use of pharmaceutically acceptable solvates of the compounds as shown herein in the manufacture of a medicament for the prevention, treatment or alleviation of pain. Therein, the solvate is hydrate, alcoholate and the like.

Regarding the use described herein, the pain is caused by cancer which includes, but is not limited to lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous cell carcinoma, basal cell carcinoma, adenocarcinoma, sweat gland carcinoma, sebaceous gland carcinoma, papillary carcinoma, papillary adenocarcinoma, adenocarcinoma cystic, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, chronic disease cells, polycythemia, lymphoma, endometrial cancer, glioma, colorectal cancer, thyroid cancer, urothelial cancer or multiple myeloma.

From the above experiments conducted by the inventor of the present application and the relevant research literature, it can be obviously known that regarding the use described herein, the pain is caused by inflammation.

Regarding the use described herein, the prepared medicament may further include other compounds or drugs used in combination with the compounds shown herein. The other compounds or drugs can also be used for the prevention, treatment or alleviation of pain.

Regarding the use described herein, the prepared medicament contains a specific dosage range of the shown compounds or salts or solvates thereof, and/or the prepared medicament is in a specific dosage form and is administered using a specific mode of administration.

The dosage of the medicament used for prevention, treatment or alleviation of pain, or the dosage of the compounds or salts or solvates thereof contained in the medicament usually depends on the specific compound applied, the patient, the specific disease or condition and the severity thereof, the route and frequency of administration and the like, and needs to be determined by the attending physician according to specific conditions. For example, when the compound or medicament provided by the present invention is administered by the oral route, the dosage may be 0.1

to 30 mg/7 days, preferably 1 to 10 mg/7 days, more preferably 5 mg/day; the dosage may be administered once every 7 days or divided into two dosages for administration twice every 7 days; preferably, the dosage is administered once every 7 days.

Regarding the use described herein, the prepared medicament may also contain pharmaceutically acceptable auxiliaries or excipients. The medicament can be any dosage form for clinical administration, such as tablets, suppositories, dispersible tablets, enteric-coated tablets, chewable tablets, orally disintegrating tablets, capsules, sugar coated agents, granules, dry powders, oral solutions, a small needle for injection, lyophilized powder for injection, or infusion solutions. According to the specific dosage form and the mode of administration, the pharmaceutically acceptable auxiliaries or excipients in the medicament may include one or more of the following: diluent, solubilizer, disintegrant, suspension, lubricant, adhesive, filler, flavoring agent, sweetener, antioxidant, surfactant, preservative, wrapping agent and pigment.

Based on the use provided in the present invention, the present invention also relates to a method for prevention, treatment or alleviation of pain, the method comprising administering an preventively, therapeutically and alleviatively effective amount of the compound of the present invention or a pharmaceutically acceptable salt or a solvate thereof to a subject in need thereof. Preferably, the subject is a mammal, more preferably a human. The pain is caused by cancer which includes, but is not limited to lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous cell carcinoma, basal cell carcinoma, adenocarcinoma, sweat gland carcinoma, sebaceous gland carcinoma, papillary carcinoma, papillary adenocarcinoma, adenocarcinoma cystic, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, chronic disease cells, polycythemia, lymphoma, endometrial cancer, glioma, colorectal cancer, thyroid cancer, urothelial cancer or multiple myeloma.

Based on the use provided in the present invention, the present invention further provides a medicament comprising the compound of formula I or formula II as defined above useful for prevention, treatment or alleviation of pain caused by cancer or inflammation.

Based on the use provided in the present invention, the present invention further provides a method for treating pain caused by cancer or inflammation, comprising a step of administering the aforementioned medicament; and a step for measuring the content of the reductase AKR1C3 of cancer cells in a patient using AKR1C3 antibodies (where the content of the AKR1C3 reductase is measured to be equal to or greater than the predetermined value, the aforementioned medicament is administered to the patient).

## Brief Description of Drawings

FIG. 1 depicts the experiment showing the effect of application of AST-3424 on the production of reduced progesterone.

## Detailed Description of the Invention

The present invention will be described below with reference to specific examples. Those skilled in the art could understand that these examples are only used for describing the invention and do not in any way limit its scope.

The experimental methods in the following examples are all conventional methods unless otherwise specified. The raw materials of the medicaments, the reagents and the like used in the following examples are all commercially available products unless otherwise specified.

The compounds shown in the present invention and the methods for synthesizing the same are disclosed in Patent Application No. PCT/US2016/021581 (WO2016/145092), Patent Application No. PCT/US2016/025665 (WO2016/161342), and Patent Application No. PCT/US2016/062114 (WO2017/087428).

The following definitions are provided to assist the reader. Unless otherwise defined, all terms of art, notations, and other scientific or medical terms or terminology used herein are intended to have the meanings commonly understood by those of skill in the chemical and medical arts. In some cases, terms with commonly understood meanings are defined herein for clarity and/or for ready reference, and the inclusion of such definitions herein should not be construed as representing a substantial difference over the definition of the term as generally understood in the art.

All numerical designations, e.g., pH, temperature, time, concentration, and weight, including ranges of each thereof, are approximations that typically may be varied (+) or (-) by increments of 0.1, 1.0, or 10.0, as appropriate. All numerical designations may be understood as preceded by the term "about". Reagents described herein are exemplary and equivalents of such may be known in the art. "$C_x$-$C_y$" or "$C_{x-y}$" before a group refers to a range of the number of carbon atoms that are present in that group. For example, $C_1$-$C_6$ alkyl refers to an alkyl group having at least 1 and up to 6 carbon atoms.

"Alkoxy" refers to -O-Alkyl.

"Amino" refers to $NR^pR^q$ wherein $R^p$ and $R^q$ independently are hydrogen or $C_1$-$C_6$ alkyl, or $R^p$ and $R^q$ together with the nitrogen atom to which they are bonded to form a 4-15 membered heterocycle.

"Aryl" refers to an aromatic group of from 6 to 14 carbon atoms and no ring heteroatoms and having a single ring (e.g., phenyl) or multiple condensed (fused) rings (e.g., naphthyl or anthryl). For multiple ring systems, including fused, bridged, and spiro ring systems having aromatic and non-aromatic rings that have no ring heteroatoms, the term "aryl" or "Ar" applies when the point of attachment is at an aromatic carbon atom (e.g., 5,6,7,8 tetrahydronaphthalene-2-yl is an aryl group as its point of attachment is at the 2-position of the aromatic phenyl ring).

According to specific embodiments of the present application, $C_6$-$C_{10}$ aryl can be phenyl, naphthyl and various substituted phenyl or naphthyl.

"Heteroaryl" refers to an aromatic group of from 1 to 14 carbon atoms and 1 to 6 heteroatoms selected from the group consisting of oxygen, nitrogen, and sulfur and includes single ring (e.g. imidazolyl-2-yl and imidazol-5-yl) and multiple ring systems (e.g. imidazopyridyl, benzotriazolyl, benzimidazol-2-yl and benzimidazol-6-yl). For multiple ring systems, including fused, bridged, and spiro ring systems having aromatic and non-aromatic rings, the term "heteroaryl" applies if there is at least one ring heteroatom, and the point of attachment is at an atom of an aromatic ring (e.g. 1,2,3,4-tetrahydroquinolin-6-yl and 5,6,7,8-tetrahydroquinolin-3-yl). In some embodiments, the nitrogen and/or the sulfur ring atom(s) of the heteroaryl group are optionally oxidized to provide for the N-oxide ($N \rightarrow O$), sulfinyl, or sulfonyl moieties. The term heteroaryl or 5-15 membered heteroaryl includes, but is not limited to, acridinyl, azocinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, benzothienyl, benzimidazolinyl, carbazolyl, NH-carbazolyl, carbolinyl, chromanyl, chromenyl, cinnolinyl, dithiazinyl, furanyl, furazanyl, imidazolidinyl, imidazolinyl, imidazopyridyl, imidazolyl, indazolyl, indolenyl, indolinyl, indolizinyl, indolyl, isobenzofuranyl, isochromanyl, isoindazolyl, isoindolinyl, isoindolyl, isoquinolinyl, isoquinolyl, isothiazolyl, isoxazolyl, naphthyridinyl, octahydroisoquinolinyl, oxadiazolyl, oxazolidinyl, oxazolyl, pyrimidinyl, phenanthridinyl, phenanthrolinyl, phenazinyl, phenothiazinyl, phenoxathiinyl, phenoxazinyl, phthalazinyl, piperazinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazole, pyridinyl, pyridyl, pyrimidinyl, pyrrolyl, quinazolinyl, quinolinyl, quinoxalinyl, quinuclidinyl, tetrahydroisoquinolinyl, tetrahydroquinolinyl, tetrazolyl, thiadiazinyl, thiadiazolyl, thianthrenyl, thiazolyl, thienyl, thienothiazolyl, thienooxazolyl, thienoimidazolyl, thiophenyl, triazinyl and xanthenyl.

"Alkyl" refers to monovalent saturated aliphatic hydrocarbyl groups having from 1 to 10 carbon atoms and, in some embodiments, from 1 to 6 carbon atoms. "$C_{x-y}$ alkyl" refers to alkyl groups having from x to y carbon atoms. This term includes, by way of example, linear and branched hydrocarbyl groups such as methyl ($CH_3$-), ethyl ($CH_3CH_2$-), n-propyl ($CH_3CH_2CH_2$-), isopropyl ($(CH_3)_2CH$-), n-butyl ($CH_3CH_2CH_2CH_2$-), isobutyl ($(CH_3)_2CHCH_2$-), sec-butyl ($(CH_3)(CH_3CH_2)CH$-), tert-butyl ($(CH_3)_3C$-), n-pentyl ($CH_3CH_2CH_2CH_2CH_2$-), and neopentyl ($(CH_3)_3CCH_2$-).

"Cycloalkyl" refers to a saturated or partially saturated cyclic group of from 3 to 14 carbon atoms and no ring heteroatoms and having a single ring or multiple rings including fused, bridged, and spiro ring systems. For multiple ring systems having aromatic and non-aromatic rings that have no ring heteroatoms, the term "cycloalkyl" applies when the point of attachment is at a non-aromatic carbon atom (e.g. 5,6,7,8-tetrahydronaphthalene-5-yl). The term "cycloalkyl" or "$C_3$-$C_8$ cycloalkyl" includes cycloalkenyl groups. Examples of cycloalkyl groups or $C_3$-$C_8$ cycloalkyl groups include, for instance, adamantyl, cyclopropyl, cyclobutyl, cyclopentyl, cyclooctyl, and cyclohexenyl.

"Heterocyclic" or "heterocycle" or "heterocycloalkyl" or "heterocyclyl" refers to a saturated or partially saturated cyclic group having from 1 to 14 carbon atoms and from 1 to 6 heteroatoms selected from the group consisting of nitrogen, sulfur, or oxygen and includes single ring and multiple ring systems including fused, bridged, and spiro ring systems. For multiple ring systems having aromatic and/or non-aromatic rings, the terms "heterocyclic", "heterocycle", "heterocycloalkyl", or "heterocyclyl" apply when there is at least one ring heteroatom, and the point of attachment is at an atom

of a non-aromatic ring (e.g. 1,2,3,4-tetrahydroquinoline-3-yl, 5,6,7,8-tetrahydroquinoline-6-yl, and decahydroquinolin-6-yl). In some embodiments, the heterocyclic groups herein are 3-15 membered, 4-14 membered, 5-13 membered, 7-12 membered, or 5-7 membered heterocycles. In some other embodiments, the heterocycles contain 4 heteroatoms. In some other embodiments, the heterocycles contain 3 heteroatoms. In another embodiment, the heterocycles contain up to 2 heteroatoms. In some embodiments, the nitrogen and/or sulfur atom(s) of the heterocyclic group are optionally oxidized to provide for the N-oxide, sulfinyl, sulfonyl moieties. Heterocyclyl includes, but is not limited to, tetrahydropyranyl, piperidinyl, N-methylpiperidin-3-yl, piperazinyl, N-methylpyrrolidin-3-yl, 3-pyrrolidinyl, 2-pyrrolidon-1-yl, morpholinyl, and pyrrolidinyl. A prefix indicating the number of carbon atoms (e.g., $C_{3-10}$) refers to the total number of carbon atoms in the portion of the heterocyclyl group exclusive of the number of heteroatoms. A divalent heterocyclic radical will have the appropriately adjusted hydrogen content.

"Ether" refers to a $C_1$-$C_6$ alkyl group substituted with 1-3 $C_1$-$C_6$ alkoxy groups, wherein alkoxy refers to -O-alkyl.

"Halo" refers to one or more of fluoro, chloro, bromo, and iodo.

"Alkenyl" refers to a linear or branched hydrocarbyl group having from 2 to 10 carbon atoms and in some embodiments from 2 to 6 carbon atoms or 2 to 4 carbon atoms and having at least 1 site of vinyl unsaturation (>C=<). For example, $C_{x-y}$ alkenyl refers to alkenyl groups having from x to y carbon atoms and is meant to include, for example, ethenyl, propenyl, 1,3-butadienyl, and the like.

"Alkynyl" refers to a linear monovalent hydrocarbon radical or a branched monovalent hydrocarbon radical having from 2 to 10 carbon atoms and in some embodiments from 2 to 6 carbon atoms or 2 to 4 carbon atoms and containing at least one triple bond. The term "alkynyl" is also meant to include those hydrocarbyl groups having one triple bond and one double bond. For example, $C_{2-6}$ alkynyl includes ethynyl, propynyl, and the like.

"Phosphoramidate alkylating agent" refers to an alkylating agent comprising one or more $Z^5$-$X^5$-$Y^5$ moieties bonded to an -O-P(Z1) moiety, where $Z^5$ is a heteroatom such as nitrogen, sulfur or oxygen, $X^5$ is optionally substituted ethylene, $Y^5$ is halo or another leaving group, or $Z^5$-$X^5$-$Y^5$ together form an aziridinyl ($NCH_2CH_2$) moiety, and $Z^1$ is defined as above. Such an alkylating agent can react with a DNA or another nucleic acid or protein. In some instances an alkylating agent can be cross-linked with DNA.

The term "optionally substituted" refers to a substituted or unsubstituted group. The group may be substituted with one or more substituents, such as e.g., 1, 2, 3, 4 or 5 substituents. Preferably, the substituents are selected from the group consisting of oxo, halo, -CN, $NO_2$, $-N_2+$, $-CO_2R^{100}$, $-OR^{100}$, $-SR^{100}$, $-SOR^{100}$, $-SO_2R^{100}$, $-NR^{100}SO_2R^{100}$, -$NR^{101}R^{102}$, $-CONR^{101}R^{102}$, $-SO_2NR^{101}R^{102}$, $C_1$-$C_6$ alkyl, $C_1$-$C_6$ alkoxy, $-CR^{100}$ = C $(R^{100})_2$, $-CCR^{100}$, $C_3$-$C_{10}$ cycloalkyl, $C_3$-$C_{10}$ heterocyclyl, $C_6$-$C_{12}$ aryl and $C_2$-$C_{12}$ heteroaryl, or a divalent substituent such as $-O-(CH^2)-O-$, $-O-(CH_2)_2-O-$, and, 1-4 methyl substituted version thereof, wherein each $R^{100}$, $R^{101}$, and $R^{102}$ independently is hydrogen or $C_1$-$C_8$ alkyl; $C_3$-$C_{12}$ cycloalkyl; $C_3$-$C_{10}$ heterocyclyl; $C_6$-$C_{12}$ aryl; or $C_2$-$C_{12}$ heteroaryl; or $R^{100}$ and $R^{102}$ together with the nitrogen atom to which they are attached to form a 5-7 membered heterocycle; wherein each alkyl, cycloalkyl, heterocyclyl, aryl, or heteroaryl is optionally substituted with 1-3 halo, 1-3 $C_1$-$C_6$ alkyl, 1-3 $C_1$-$C_6$ haloalkyl or 1-3 $C_1$-$C_6$ alkoxy groups. Preferably, the substituents are selected from the group consisting of chloro, fluoro, $-OCH_3$, methyl, ethyl, iso-propyl, cyclopropyl, - $CO_2H$ and salts and $C_1$-$C_6$ alkyl esters thereof, $CONMe_2$, $CONHMe$, $CONH_2$, $-SO_2Me$, $-SO_2NH_2$, $-SO_2NMe_2$, $-SO_2NHMe$, $-NHSO_2Me$, $-NHSO_2CF_3$, $-NHSO_2CH_2Cl$, $-NH_2$, - $OCF_3$, $-CF_3$ and $-OCHF_2$.

"Alkylene" refers to divalent saturated aliphatic hydrocarbyl groups having from 1 to 10 carbon atoms and, in some embodiments, from 1 to 6 carbon atoms. "$C_{u-v}$ alkylene" refers to alkylene groups having from u to v carbon atoms. The alkylidene and alkylene groups include branched and straight chain hydrocarbyl groups. For example, "$C_{1-6}$ alkylene" includes methylene, ethylene, propylene, 2-methylpropylene, pentylene, and the like.

"Heteroalkylene" refers to an alkylene wherein a chain carbon atom is replaced with a heteroatom such as O, S, N, or P, or a heteroatom containing substituent.

The "drugs" regarding D herein includes without limitation, gemcitibine, erlotinib, meturedepa, uredepa, altretamine, imatinib, triethylenemelamine, trimethylmelamine, chlorambucil, chlornaphazine, estramustine, gefitinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, mannomustine, mitobronitol, mitolactol, pipobroman, aclacinomycins, actinomycin, anthramycin, azaserine, bleomycin, cactinomycin, carubicin, carzinophilin, chromomycin, dactinomycin, daunorubicin, daunomycin, 6-diazo-5-oxo-1-norleucine, mycophenolic acid, nogalamycin, olivomycin, peplomycin, plicamycin, porfiromycin, puromycin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine, 5-fluorouracil, tegafur, L-asparaginase, pulmozyme, aceglatone, aldophosphamide glycoside, aminolevulinic acid, amsacrine, bestrabucil, bisantrene, defofamide, demecolcine, diaziquone, elfornithine, elliptinium acetate, etoglucid, flutamide, hydroxyurea, interferon-alpha, interferon-beta, interferon-gamma, interleukin-2, lentinan, mitoguazone, mitoxantrone, mopidamol, nitracrine, pentostatin, phenamet, pirarubicin, podophyllinic acid, 2-ethylhydrazide, procarbazine, razoxane, sizofiran, spirogermanium, paclitaxel, tamoxifen, erlotonib, teniposide, tenuazonic acid, triaziquone, 2,2',2"-trichlorotriethylamine, urethan, vinblastine, and vincristine.

"Administering" or "administration of' a drug to a patient (and grammatical equivalents of this phrase) refers to direct administration, which may be administered to a patient by a medical professional or may be self-administered, and/or indirect administration, which may be the act of prescribing a drug. For example, a physician who instructs a patient to self-administer a drug and/or provides a patient with a prescription for a drug is administering the drug to the patient.

"Cancer" refers to leukemias, lymphomas, carcinomas, and other malignant tumors, including solid tumors, of potentially unlimited growth that can expand locally by invasion and spread throughout the bodyby metastasis. Examples of cancers include, but are not limited to, cancer of the adrenal gland, bone, brain, breast, bronchi, colon and/or rectum, gallbladder, head and neck, kidneys, larynx, liver, lung, neural tissue, pancreas, prostate, parathyroid, skin, stomach, and thyroid. Certain other examples of cancers include, acute and chronic lymphocytic and granulocytic tumors, adenocarcinoma, adenoma, basal cell carcinoma, poor cervical intraepithelial differentiation and in situ carcinoma, Ewing's sarcoma, epidermoid carcinomas, giant cell tumor, glioblastoma multiforma, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary epithelial carcinoma, metastatic skin carcinoma, mucosal neuroma, myeloma, mycosis fungoides, neuroblastoma, osteo sarcoma, osteogenic and other sarcoma, ovarian tumor, pheochromocytoma, polycythermia vera, primary brain tumor, small-cell lung tumor, squamous cell carcinoma of both ulcerating and papillary type, hyperplasia, seminoma, soft tissue sarcoma, retinoblastoma, rhabdomyosarcoma, renal cell tumor, topical skin lesion, reticulum cell sarcoma, and Wilm's tumor.

"Inflammation" is preferably the inflammation which causes pain due to the prostaglandin E2/F2 from the above-mentioned arachidonic acid-cyclooxygenase - prostaglandin pathway.

"Patient" and "subject" are used interchangeably to refer to a mammal in need of treatment for cancer. Generally, the patient is a human. Generally, the patient is a human diagnosed with cancer. In certain embodiments, a "patient" or "subject" may refer to a non-human mammal used in screening, characterizing, and evaluating drugs and therapies, such as, a non-human primate, a dog, cat, rabbit, pig, mouse or a rat.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392, incorporated herein by reference). A prodrug may be synthesized using reactants other than the corresponding drug.

"Solid tumor" refers to solid tumors including, but not limited to, metastatic tumors in bone, brain, liver, lungs, lymph node, pancreas, prostate, skin and soft tissue (sarcoma).

"Therapeutically effective amount" of a drug refers to an amount of a drug that, when administered to a patient with cancer, will have the intended therapeutic effect, e.g., alleviation, amelioration, palliation or elimination of one or more manifestations of cancer in the patient. A therapeutic effect does not necessarily occur by administration of one dose, and may occur only after administration of a series of doses. Thus, a therapeutically effective amount may be administered in one or more administrations.

"Treatment of" a condition or patient refers to taking steps to obtain beneficial or desired results, including clinical results. For purposes of this invention, beneficial or desired clinical results include, but are not limited to, alleviation or improvement of one or more symptoms of cancer; diminishment of extent of disease; delay or slowing of disease progression; alleviation, palliation, or stabilization of the disease state; or other beneficial results. Treatment of cancer may, in some cases, result in partial response or stable disease.

"Tumor cells" refers to tumor cells of any appropriate species, e.g., mammalian such as murine, canine, feline, equine or human.

The above description of embodiments of the present invention does not limit the present invention. Those skilled in the art can make various modifications and changes according to the present invention, and any modification and change within the spirit of the present invention shall be covered in the scope of the claims appended to the present invention.

An *in vitro* experiment and an *in vivo* experiment in cynomolgus monkeys are provided below to verify the analgesic effects of the compounds.

*In vitro* experiments

Experimental apparatus: Waters Acquity I Class UPLC Ultra Performance Liquid Chromatograph equipped with a Xevo G2-XS Q Tof HRMS Quadrupole Time-of-flight High-Resolution Mass Spectrometer

Buffers and materials:

1. PBS phosphate buffered saline solution,
2. PBS phosphate buffered saline solution of 20 mM NADPH
3. PBS phosphate buffered saline solution of 250 μg/mL AKR1C3
4. 50% MeOH/H2O solution of 250 μM AST-3424
5. 50% MeOH/H2O solution of 250 μM progesterone
6. 100% acetonitrile solution of 1 μg/mL propranolol

Experimental procedures

In step 1, the reaction mixtures were prepared into Eppendorf tubes in quadruplicate (n=4) according to the table below and mixed gently.

| Materials | **Negative controls (μL)** | **Samples (μL)** |
|---|---|---|
| PBS | 68 | 58 |
| NADPH (20 mM) | 10 | 10 |
| AKR1C3 (250 μg/mL) | 10 | 10 |
| AST-3424 (250 μM) | 0 | 10 |

In step 2, the above mixtures were pre-incubated in duplicate at 37°C for 30 minutes and 60 minutes.
In step 3, another 10 μL of PBS phosphate buffered saline solution of 20 mM NADPH and 2 μL of 50% MeOH/$H_2O$ solution of 250 μM progesterone were added to each Eppendorf tube and mixed gently.
In step 4, 50 μL of the mixture in the above step was immediately transferred to 100 μL of 100% acetonitrile solution of 1 μg/mL propranolol (internal standard (IS)).
In step 5, the remaining samples were incubated at 37°C for 30 minutes, and 100 μL of 100% acetonitrile solution of 1 μg/mL propranolol (internal standard (IS)) was added.
In step 6, 100 μL of reagent water was added to all the samples, vortexed at 1,100 rpm for 5 minutes, and centrifuged at 15,000 rpm for 10 minutes at room temperature.
In step 7, all the samples were loaded on LC/MS to determine the content of reduced progesterone, namely 20α-dihydroprogesterone.
The test conditions for the LC-MS apparatus are shown below

| **Items** | **Conditions** |
|---|---|
| **Apparatus:** | Waters Acquity I Class Liquid Chromatograph |
| **Chromatographic column:** | Acquity UPLC BEHC18 Chromatographic Column (50*2.1 mm, 1.7 μm) |
| **Flow rate:** | 0.4 mL/min |
| **Injection volume:** | 3 μL |
| **The composition of the mobile phase:** | A: 0.1% (V/V) formic acid aqueous solution<br>B: 0.1% (V/V) formic acid acetonitrile solution |
| **The temperature of the column oven:** | 40°C |
| **Detector:** | Quadrupole Time-of-flight Mass Spectrometer Q-TOF MS |

The gradient of elution of the liquid phase

| Time (min) | A (%) | B (%) |
|---|---|---|
| 0.00 | 90.0 | 0.0 |
| 1.5 | 5.0 | 95.0 |
| 2.00 | 5.0 | 95.0 |
| 2.30 | 90.0 | 10.0 |

(continued)

| Time (min) | A (%) | B (%) |
|---|---|---|
| 3.00 | 90.0 | 10.0 |

Parameters of the Quadrupole Time-of-flight Mass Spectrometer

| **Items** | **Parameters** |
|---|---|
| **Capillary voltage (kV)** | 2.5 |
| **Cone voltage (V)** | 40 |
| **Source temperature (°C)** | 100 |
| **Flow rate of cone gas (L/h)** | 50 |
| **Flow rate of desolvation gas (L/h)** | 600 |
| **Ionization mode (Interface type)** | ES, Positive |
| **Analyser mode** | Sensitivity |
| **Scan range** | 50-800 m/z |

In step 9, the reduced progesterone (20α-dihydroprogesterone) is calculated: the peak area of the reduced progesterone, namley 20α-dihydroprogesterone and propranolol, in each sample was determined by LC/MS. The peak area ratios of reduced progesterone to propranolol (i.e., the ratios in the above table) were calculated, and when the time is 0 the ratio is set to 0%.

AKR1C3 activity (%) = [(the amount of reduced progesterone after normalization of the sample) 30min - (the amount of reduced progesterone after normalization of the sample) 0 min] / [(the amount of reduced progesterone after normalization of the negative control group) 30min - (the amount of reduced progesterone after normalization of the negative control group) 0 min] * 100.

The AKR1C3 activity results in the above table were calculated according to the above formula.

Experimental results

| | Duration | | The peak areas of the liquid phase | | | | | | | | AKR1C3 activity % |
|---|---|---|---|---|---|---|---|---|---|---|---|
| | Incubation | Reaction | AST-3424 | Progesterone | | Reduced progesterone | | | **Propranolol** | | |
| | | | | Values | Averages | Values | Averages | Ratios * | Values | Averages | |
| Negative control groups | 30 min | 0 | Absent | 36658 | 36835 | 150 | 158 | 0.0034 | 51141 | 46879 | 0 |
| | | | | 37011 | | 166 | | | 42616 | | |
| | 60 min | | | 35244 | 35125 | 87 | 83 | 0.0019 | 42794 | 43650 | 0 |
| | | | | 35006 | | 79 | | | 44506 | | |
| | 30 min | 30 min | | 28675 | 26228 | 5616 | 6617 | 0.1506 | 43589 | 43935 | 100 |
| | | | | 23780 | | 7617 | | | 44280 | | |
| | 60 min | | | 25917 | 26492 | 6747 | 6051 | 0.1343 | 44258 | 45067 | 100 |
| | | | | 27067 | | 5354 | | | 45876 | | |
| Samples | 30 min | 0 | 154017 | 37107 | 37438 | 70 | 72 | 0.0017 | 43247 | 43436 | 0 |
| | | | 154843 | 37769 | | 74 | | | 43625 | | |
| | 60 min | | 108351 | 38050 | 37989 | 58 | 64 | 0.0015 | 43907 | 43697 | 0 |
| | | | 107508 | 37928 | | 70 | | | 43487 | | |
| | 30 min | 30 min | 104748 | 35187 | 35210 | 344 | 331 | 0.0074 | 45657 | 45051 | 3.9 |
| | | | 110630 | 35234 | | 319 | | | 44445 | | |
| | 60 min | | 52296 | 32636 | 32520 | 622 | 616 | 0.0137 | 45395 | 45101 | 9.2 |
| | | | 51309 | 32404 | | 610 | | | 44807 | | |

Analysis and summary of the experimental results

Table: The effect of AST-3424 on AKR1C3 activity

| | **% AKR1C3 activity** | |
|---|---|---|
| The time of incubation (min) | 0 μM AST-3424 | 5 μM AST-3424 |
| 30 | 100% | 3.9% |
| 60 | 100% | 9.2% |

The effect of AST-3424 on the production of reduced progesterone is shown in FIG. 1. The aforementioned *in vitro* experiment proves that after pre-incubation for 30 minutes and 60 minutes, AST-3424 at a concentration of 5 μM basically inhibited AKR1C3 activity: compared with the negative control groups, the production of the reduced progesterone, namely 20α-dihydroprogesterone, was reduced to 3.9% and 9.2%, respectively, proving that the compound AST-3424 and the similar compounds disclosed in the patent applications No. PCT/US2016/021581, PCT/US2016/025665 and PCT/US2016/062114 are inhibitors of AKR1C3 enzyme.

**II. *In vivo* experiment**

An experiment with three cynomolgus monkeys was performed according to the table below.

| Group | Amount | Administration | | | | | |
|---|---|---|---|---|---|---|---|
| | Male | Test substance | The dosage of the test substance (mg/kg) | The concentration of the solution of the test substance (mg/mL) | The volume of administration (mL/kg) | Mode of administration | Collected Sample |
| 1 | 3 | AST3424 | 1/Compound | 0.2 | 5 | Intravenous infusion | Plasma/serum |

Four male cynomolgus monkeys were purchased from Guangxi Xiongsen Primate Development and Experiment Co., Ltd., and all of them were healthy cynomolgus monkeys that had passed the physical examination and have no abnormalities. Among them, three cynomolgus monkeys were used in the experiment of administration and the remaining one was used for preparing blank plasma.

Before administration, and 6, 24, 48 and 72 hours after the start of administration, 1 mL of blood was collected from the femoral vein or other suitable vein, and placed in a blood collection tube without anticoagulant. After collected, the blood sample was placed on ice and allowed to stand for 30-60 minutes before being centrifuged at 3,500 rpm for 10 minutes at 2-8 °C to separate the serum. The collected serum was stored at -80°C before being analyzed.

Prostaglandins E2 and F2 in serum samples were analyzed by conventional ELISA methods. The measurement results are as follows.

The concentrations of prostaglandins E2 and F2 in the serum after single administration to cynomolgus monkeys by intravenous infusion

| **Time point** | **The measured concentration** of prostaglandin F2 **(pg/ml)** | | | | |
|---|---|---|---|---|---|
| | **101** | **102** | **103** | **Mean** | **SD** |
| Before administration | 1828.51 | 310.17 | 1125.58 | 1088.09 | 759.86 |
| 6h | 165.32 | 260.80 | 300.65 | 242.26 | 69.54 |
| 24h | 816.63 | 3460.25 | 4208.19 | 2828.36 | 1781.89 |
| 48h | 183.73 | 216.12 | 541.05 | 313.63 | 197.61 |
| 72h | 441.76 | 968.01 | 1369.67 | 926.48 | 465.35 |
| **Time point** | **The measured concentration** of prostaglandin E2 **(pg/ml)** | | | | |
| | **101** | **102** | **103** | **Mean** | **SD** |
| Before administration | 713.91 | 233.63 | 461.26 | 469.60 | 240.25 |
| 6h | 210.67 | 214.73 | 670.73 | 365.38 | 264.45 |
| 24h | 450.48 | 822.21 | 2735.20 | 1335.97 | 1225.95 |
| 48h | 322.13 | 168.84 | 276.35 | 255.78 | 78.69 |
| 72h | 408.15 | 521.78 | 663.03 | 530.99 | 127.69 |

After 0.58 mg/kg of AST3424 was administered to cynomolgus monkeys, both prostaglandins E2 and F2 decreased; they fluctuatingly increased at 24h, which was speculated to be related to the time characteristics of the secretion of prostaglandins E2 and F2 by the cynomolgus monkeys themselves. This demonstrates that AST3424 can inhibit the secretion of prostaglandins E2 and F2 by cynomolgus monkeys.

The causes of pain directly arising from a tumor and bone infiltration and metastasis include the following: direct bone involvement and direct activation of local nociceptors; compression of adjacent nerves, blood vessels and soft tissues by the tumor; and release of PGE1 and PGF2 from bone infiltration by the tumor. PGF2 is a strong pain-causing factor. The aforementioned animal experiment shows that the AST-3424 compound can greatly reduce the content of PGF2, thereby achieving the effect of treating/alleviating the pain caused by cancer or tumor.

### III. Experiment conclusion

The above *in vitro* and *in vivo* experiments verify that the aforementioned specific substrate, namely DNA alkylating agent targeting overexpressed aldo-keto reductase AKR1C3, can inhibit the activity of the aldo-keto reductase AKR1C3. Moreover, the animal experiment proves that the specific substrates can reduce the content of prostaglandin E2/F2 in the blood. Accordingly, it can be proved that the aforementioned compound AST-3424 and the similar compounds disclosed in the patent applications No. PCT/US2016/021581, PCT/US2016/025665 and PCT/US2016/062114 are inhibitors of AKR1C3 enzyme, which can block the production of prostaglandin E2/F2, reduce its content, and has an analgesic effect.

## Claims

1. Use of a compound of formula I or a pharmaceutically acceptable salt, or a solvate thereof in the manufacture of a medicament for preventing, treating or alleviating pain,

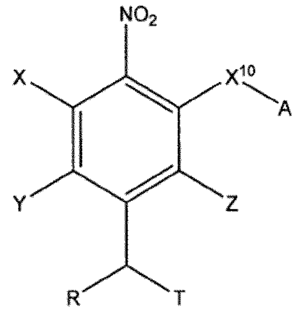

I

wherein

$X^{10}$ is O, S, SO, or $SO_2$;
A is $C_6$-$C_{10}$ aryl or substituted aryl, 5-15 membered heteroaryl or substituted heteroaryl, or $-N=CR^1R^2$, wherein each $R^1$ and $R^2$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;
each X, Y, and Z independently is hydrogen, CN, halo, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;
R is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;
each $R^{13}$ and $R^{14}$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl or ether, or $R^{13}$ and $R^{14}$ together with the nitrogen atom to which they are bonded to form 5-7 membered heterocyclyl group;
T comprises a phosphoramidate alkylating agent; and
wherein the alkyl, alkenyl, alkynyl, cycloalkyl, aryl, heterocycle, heteroaryl, ether groups are optionally substituted.

2. The use according to claim 1, wherein in the compound of formula I, T is $OP(Z^1)(NR^{30}CH_2CH_2X^1)_2$, $OP(Z^1)(NR^{30}_2)(N(CH_2CH_2X^1)_2)$, $OP(Z^1)(N(CH_2)_2)_2$ or $OP(Z^1)(N(CH_2CH_2X^1)_2)_2$, wherein each $R^{30}$ independently is hydrogen or $C_1$-$C_6$ alkyl or two $R^{30}$ groups together with the nitrogen atom to which they are bonded to form 5-7 membered heterocyclyl group, $Z^1$ is O or S, and $X^1$ is Cl, Br, or OMs or other leaving groups.

3. The use according to claim 1, wherein in the compound of formula I, T is $OP(Z^1)(NHCH_2CH_2Cl)_2$, $OP(Z^1)(NHCH_2CH_2Br)_2$, $OP(Z^1)(NH_2)(N(CH_2CH_2X^1)_2)$, $OP(Z^1)(N(CH_2)_2)_2$ or $OP(Z^1)(N(CH_2CH_2Cl)_2)_2$, wherein $Z^1$ is O or S, and $X^1$ is Cl, Br, or OMs.

4. The use according to claim 1, wherein in the compound of formula I, T is $OP(O)(N(CH_2CH_2))_2$, $OP(O)(NHCH_2CH_2Cl)_2$, $OP(O)(NHCH_2CH_2Br)_2$ or $OP(O)(NH_2)(N(CH_2CH_2Cl)_2)$.

**5.** The use according to claim 1, wherein the compound of formula I is

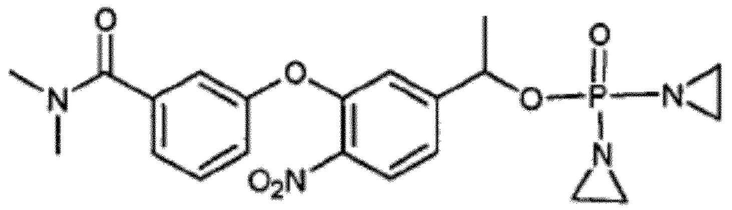

,

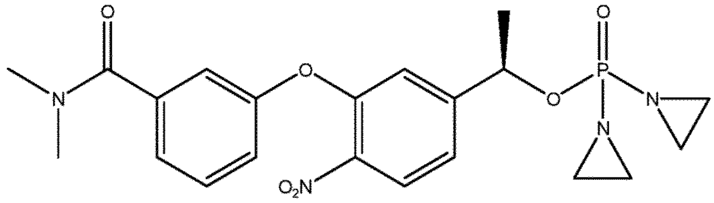

,

or

5

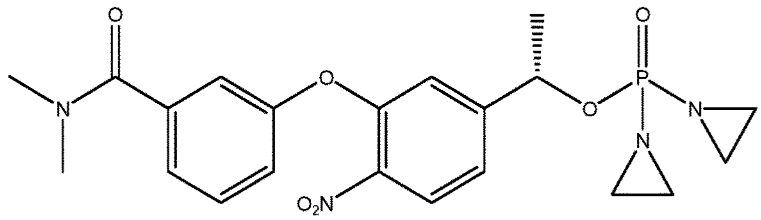

.

**6.** Use of a compound of formula II or a pharmaceutically acceptable salt, or a solvate thereof in the manufacture of a medicament for the prevention, treatment or alleviation of pain,

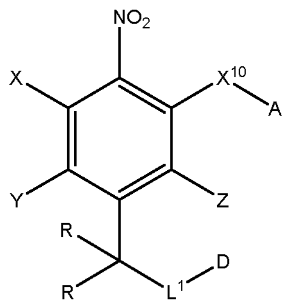

II

wherein

$X^{10}$ is O, S, SO, or $SO_2$;
A is $C_6$-$C_{10}$ aryl or substituted aryl, 5-15 membered heteroaryl or substituted heteroaryl, or $-N=CR^1R^2$, wherein each $R^1$ and $R^2$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;
each X, Y, and Z independently is hydrogen, CN, halo, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$, or $-NR^{13}COR^{14}$;
each R independently is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, - $CONR^{13}R^{14}$, or $-NR^{13}COR^{14}$;
each $R^{13}$ and $R^{14}$ independently is hydrogen, $C_1$-$C_6$ alkyl, $C_3$-$C_8$ cycloalkyl, $C_6$-$C_{10}$ aryl, 4-15 membered heterocycle, 5-15 membered heteroaryl, or ether, or $R^{13}$ and $R^{14}$ together with the nitrogen atom to which they are

bonded to form 5-7 membered heterocyclyl group;

$L^1$ and D are as defined in the specification and have the following specific definitions:

$L^1$ is selected from:

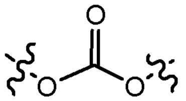,
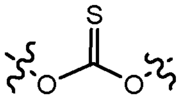,
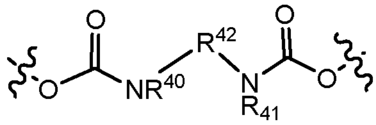,
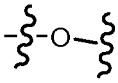,

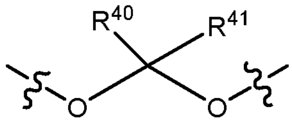 and 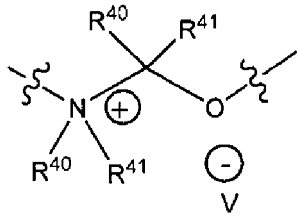 ;

wherein $R^{40}$ and $R^{41}$ are independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, or 5-15 membered heteroaryl;

$R^{42}$ is $C_2$-$C_3$ alkylene or heteroalkylene optionally substituted with 1-3 $C_1$-$C_6$ alkyl; V(-) is any anion, preferably, a pharmaceutically acceptable anion; and

D is a moiety such that D-OH is an anticancer drug wherein OH is an aliphatic or a phenolic hydroxy group or is an OH moiety attached to a phosphorous atom as provided herein; in other words, D is the remaining group in the anticancer drug DOH after the hydroxyl group is removed therefrom;

alternatively

$L^1$ is:

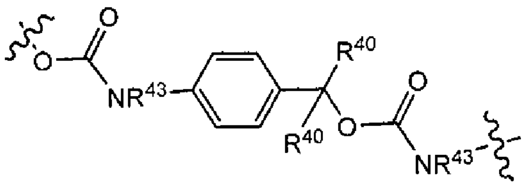,
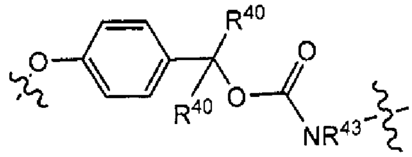

or

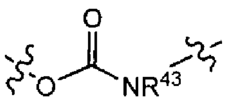;

wherein $R^{40}$ is defined as above, $R^{43}$ is hydrogen or together with D forms a heterocycle, and the phenyl moiety is optionally substituted, and

D is a moiety such that D-$NR^{43}$H is an anticancer drug; in other words, D is the remaining group in the anticancer drug D-$NR^{43}$H after amino or amine is removed therefrom;

alternatively

$L^1$ is a bond, -O-$C(R^{40}R^{41})_2$-, -O-$C(R^{40}R^{41})$-$NR^{40}R^{41}$(+)-$C(R^{40}R^{41})$- or

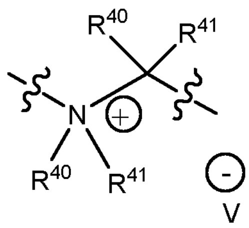
,

wherein $R^{40}$, $R^{41}$ and V are defined as above, and
D is an anticancer drug containing a primary or a secondary amine, wherein the primary or the secondary amine is bonded to $L^1$; and
wherein the alkyl, alkenyl, alkynyl, cycloalkyl, aryl, heterocycle, heteroaryl, and ether groups are optionally substituted.

**7.** The use according to claim 6, wherein the compound II is of formula II-A,

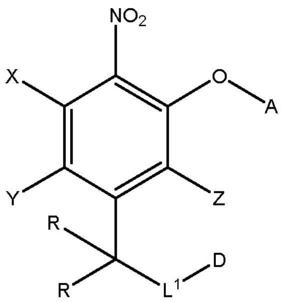
or
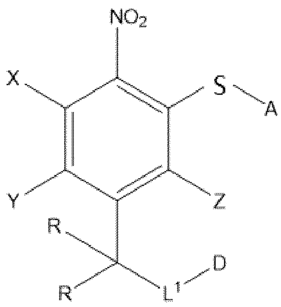

II-A

wherein the remaining variables are as defined in claim 6.

**8.** The use according to claim 6, wherein the compound II is of formula IIA-1:

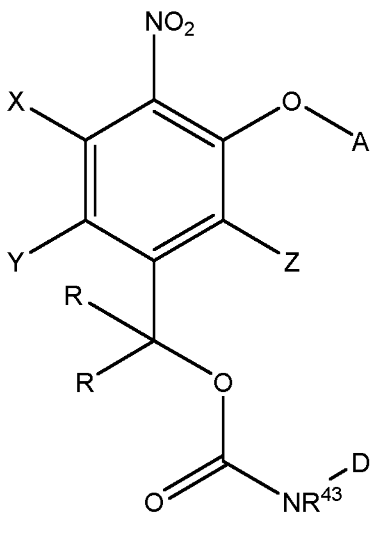
or
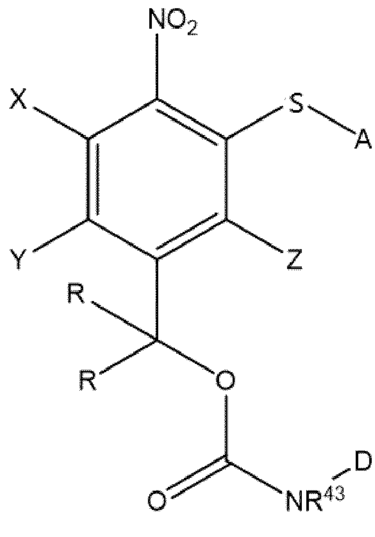

IIA-1

wherein the remaining variables are as defined in claim 6, and D is a moiety of the cytotoxic agent $HNR^{43}$-D containing a primary or a secondary amine group; in other words, D is the remaining group in the cytotoxic agent $HNR^{43}$-D after the primary or a secondary amine group is removed therefrom;

alternatively,
the compound is of formula III-2 or IIIA-3:

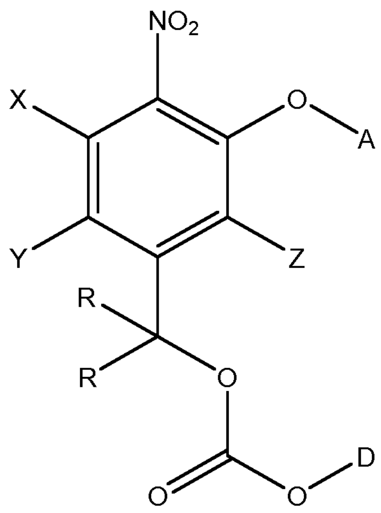
or
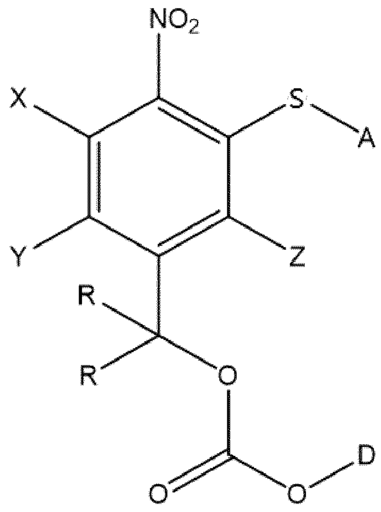

IIA-2

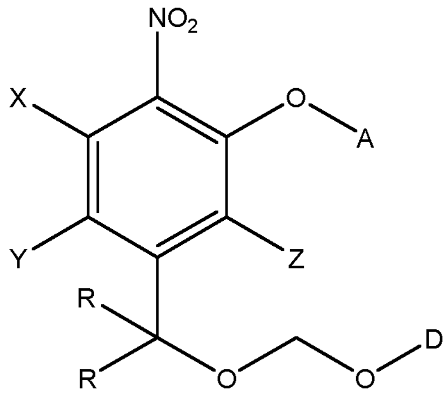
or
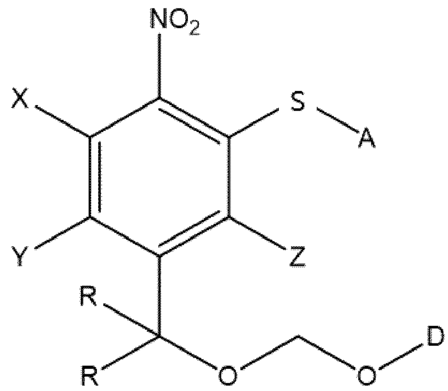

IIA-3

wherein the remaining variables are as defined in claim 6, and D is a moiety of the cytotoxic agent HO-D containing at least one hydroxyl group; in other words, D is the remaining group in the cytotoxic agent HO-D after the hydroxyl group is removed therefrom;
alternatively,
the compound is of IIA-4, IIA-6 or IIA-6-i:

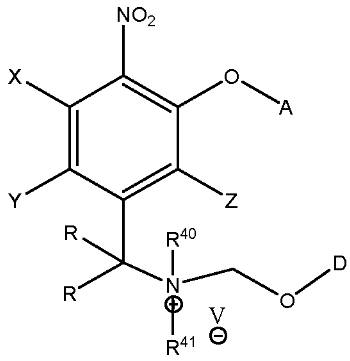

or

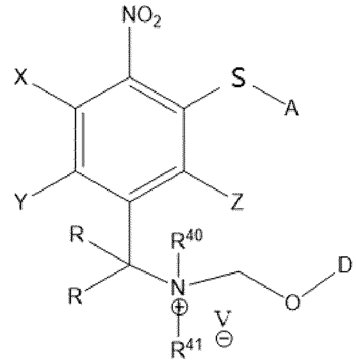

IIA-4

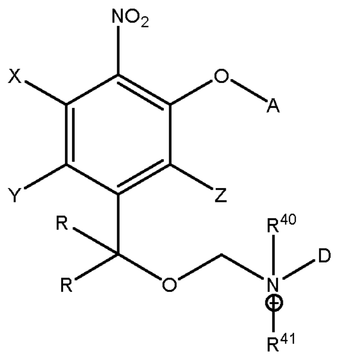

or

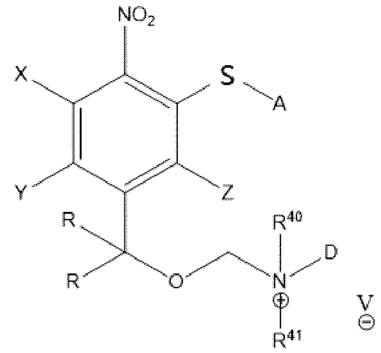

IIA-6-i

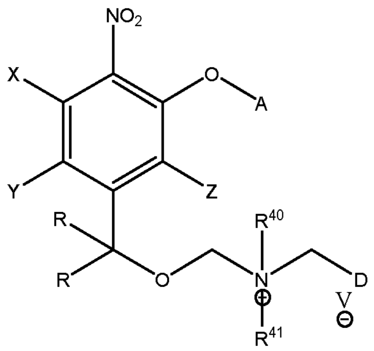

or

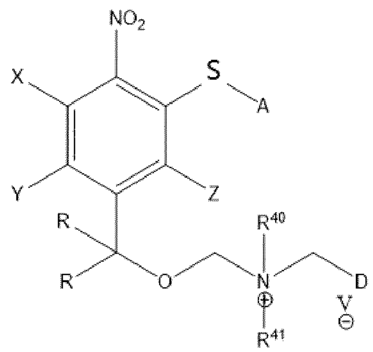

,

IIA-6

wherein the variables are as defined in claim 6; wherein in IIA-4, HO-D is cytotoxic agent containing at least one hydroxyl group; in other words, D is a moiety of the cytotoxic agent HO-D containing at least one hydroxyl group, or D is the remaining group in the cytotoxic agent HO-D after the hydroxyl group(s) is/are removed therefrom; in IIA-6-i, $DNR^{40}R^{41}$ is a drug; in other words, D is a moiety such that $DNR^{40}R^{41}$ is an anticancer drug or D is the remaining moiety in the anticancer $DNR^{40}R^{41}$ after $NR^{40}R^{41}$ is removed therefrom; and in IIA-6, D is a drug containing a secondary amine, wherein the secondary amine is bonded to the methylene group; in other words, D is a drug containing a secondary amine, and is bonded via the secondary amine contained therein to the methylene group so as to be linked to - $NR^{40}R^{41}$ as shown in the above formula IIA-4, IIA-6 or IIA-6-I; alternatively,

the compound is of IIA-5 or IIA-7:

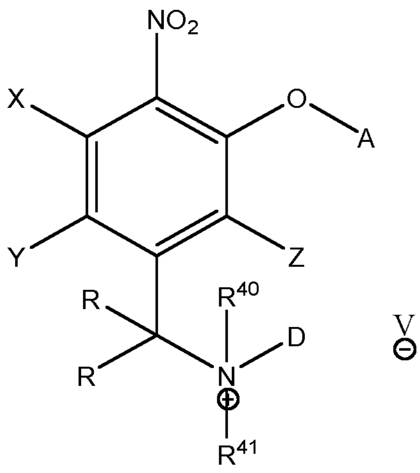

or

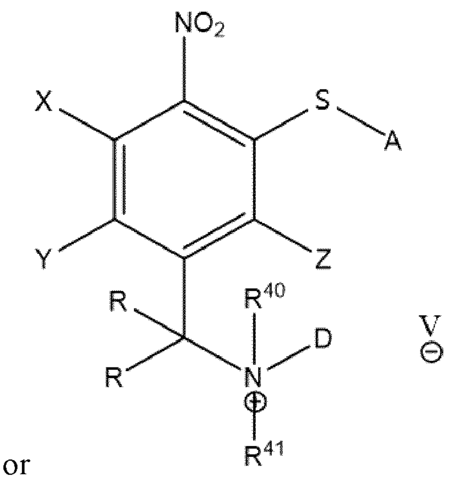

IIA-5

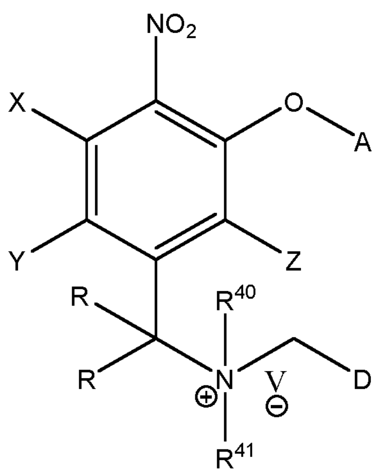

or

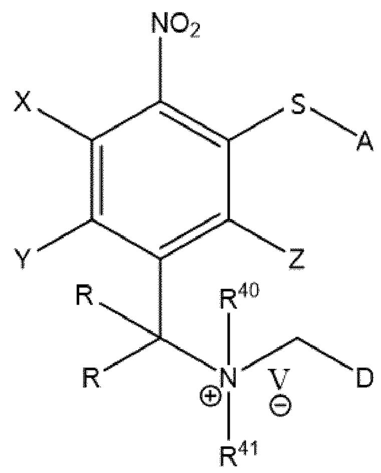

,

IIA-7

wherein the variables are as defined in claim 6; wherein in IIA-5, $DNR^{40}R^{41}$ is a drug; in other words, D is a moiety such that $DNR^{40}R^{41}$ is an anticancer drug or D is the remaining moiety in the anticancer $DNR^{40}R^{41}$ after $NR^{40}R^{41}$ is removed therefrom; and IIA-7, D is a drug containing a secondary amine, wherein the secondary amine is bonded to the methylene group as shown in the above formula IIA-5 or IIA-7.

**9.** The use according to any of claims 6-8, wherein in the compound of formula II, Z is hydrogen, or X is hydrogen, or Y is hydrogen/halo.

**10.** The use according to any of claims 6-8, wherein in the compound of formula II, A is substituted or unsubstituted $C_6$-$C_{10}$ aryl, or phenyl, or 5-15 membered heteroaryl, or pyridyl, or $-N=CR^1R^2$, wherein $R^1$ and $R^2$ are as defined in claim 6.

**11.** The use according to any of claims 6-8, wherein in the compound of formula II, each R is hydrogen, or one of the R groups is hydrogen and the other R group is $C_1$-$C_6$ alkyl, or both R groups are non-hydrogen substituents as defined in claim 6, or R is methyl.

**12.** The use according to any of claims 6-8, wherein in the compound of formula II, each of $R^{40}$, $R^{41}$ and $R^{43}$ is independently hydrogen or methyl and $R^{42}$ is $-CH_2-CH_2-$ or $CH_2-C(Me)_2-$.

**13.** The use according to any of claims 6-8, wherein in the compound of formula II, D excludes a phosphoramidate alkylating agent such as $-P(Z^1)(NR^{30}CH_2CH_2X^1)_2$, $-P(Z^1)(NR^{30}{}_2)(N(CH_2CH_2X^1)_2)$, $-P(Z^1)(N(CH_2CH_2))_2$ or $-P(Z^1)(N(CH_2CH_2X^1)_2)_2$, wherein each $R^{30}$ independently is hydrogen or $C_1$-$C_6$ alkyl or 2 $R^{30}$s together with the nitrogen atom to which they are bonded to form 5-7 membered heterocyclyl group, $Z^1$ is O or S, and $X^1$ is Cl, Br, or OMs or another leaving group.

**14.** The use according to any of claims 1-13, wherein the salt is a basic salt or an acid salt.

**15.** The use according to any of claims 1-3, wherein the solvate is hydrate or alcoholate.

**16.** The use according to any of claims 1-13, wherein the pain is a pain caused by cancer or inflammation.

**17.** A medicament comprising the compound of formula I or formula II as defined in claims 1-13 for prevention, treatment or alleviation of pain caused by cancer or inflammation.

**18.** A method for treatment of pain caused by cancer or inflammation, the method comprising a step of administering the medicament of claim 18; and a step for measuring the content of AKR1C3 reductase of cancer cells in a patient using AKR1C3 antibodies, where the content of AKR1C3 reductase is measured to be equal to or greater than the predetermined value, the medicament of claim 18 is administered to the patient.

Fig. 1

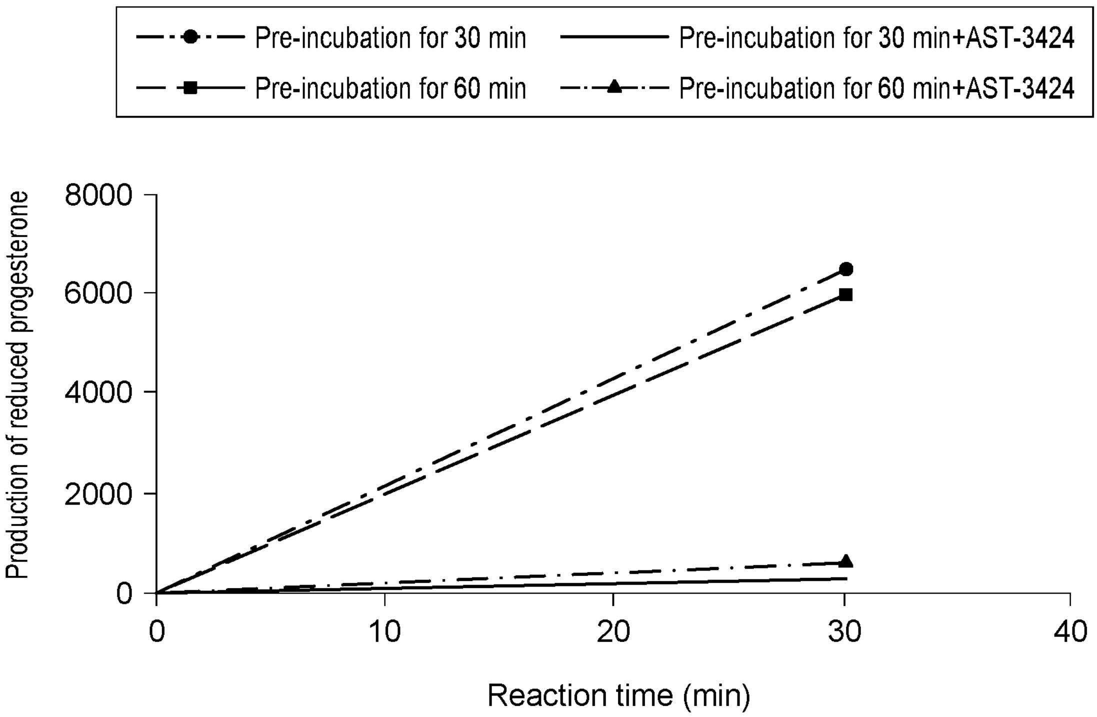
Pre-incubation for 30 min
Pre-incubation for 30 min+AST-3424
Pre-incubation for 60 min
Pre-incubation for 60 min+AST-3424
Production of reduced progesterone
8000
6000
4000
2000
0
0
10
20
30
40
Reaction time (min)

**INTERNATIONAL SEARCH REPORT**

International application No.
**PCT/CN2019/084604**

**A. CLASSIFICATION OF SUBJECT MATTER**

A61K 31/04(2006.01)i; A61K 31/06(2006.01)i; A61K 31/664(2006.01)i; C07C 205/38(2006.01)i; C07F 9/564(2006.01)i; C07B 55/00(2006.01)i; C07F 9/22(2006.01)i; A61P 29/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CPRSABS, CNABS, CNTXT, CNKI, 万方数据库, WANFANG DATABASE, 百度学术, BAIDU SCHOLAR, DWPI, SIPOABS, WOTXT, USTXT, EPTXT, ISI WEB OF KNOWLEDGE, STN: 醛酮还原酶, AKR1C3, 抑制剂, 疼, 痛, 通式I, II的化合物, aldo-keto reductase, inhibitor, ache, pain

**C. DOCUMENTS CONSIDERED TO BE RELEVANT**

| Category* | Citation of document, with indication, where appropriate, of the relevant passages | Relevant to claim No. |
|---|---|---|
| A | CN 107530556 A (THRESHOLD PHARMACEUTICALS, INC.) 02 January 2018 (2018-01-02)<br>claims 1-20 | 1-18 |
| A | CN 108136214 A (THRESHOLD PHARMACEUTICALS, INC.) 08 June 2018 (2018-06-08)<br>claims 1-20 | 1-18 |
| A | WO 2017087428 A1 (THRESHOLD PHARMACEUTICALS, INC.) 26 May 2017 (2017-05-26)<br>claims 1-11 | 1-18 |
| A | CN 103827133 A (BAYER INTELLECTUAL PROPERTY GMBH; BAYER PHARMA AKTIENGESELLSCHAFT) 28 May 2014 (2014-05-28)<br>claims 1-8, and description, paragraph 0127 | 1-18 |
| A | US 5424424 A (SEARLE & CO.) 13 June 1995 (1995-06-13)<br>claims 1-20 | 1-18 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 July 2019** | **31 July 2019** |

| Name and mailing address of the ISA/CN | Authorized officer |
|---|---|
| **China National Intellectual Property Administration**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT

International application No.
**PCT/CN2019/084604**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18**
because they relate to subject matter not required to be searched by this Authority, namely:

[1] Claim 18 relates to a method for the treatment of the pain caused by cancer or inflammation, which falls within the subject matter for which a search is not required (PCT Rule 39.1(iv)). However, it is expected that the applicant will amend claim 18 to a pharmaceutical use claim, and hence the international search report has been made on this basis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/084604**

| Patent document cited in search report | Publication date (day/month/year) | Patent family member(s) | Publication date (day/month/year) |
|---|---|---|---|
| CN 107530556 A | 02 January 2018 | EP 3277380 A4 | 01 August 2018 |
| | | SG 11201707293V A | 30 October 2017 |
| | | WO 2016145092 A1 | 15 September 2016 |
| | | AU 2016229136 B2 | 09 August 2018 |
| | | KR 20170128280 A | 22 November 2017 |
| | | IL 254377 D0 | 30 November 2017 |
| | | BR 112017019287 A2 | 02 May 2018 |
| | | JP 2018513876 A | 31 May 2018 |
| | | AU 2016229136 A1 | 05 October 2017 |
| | | US 2018044360 A1 | 15 February 2018 |
| | | TW 201706262 A | 16 February 2017 |
| | | EP 3277380 A1 | 07 February 2018 |
| | | CA 2979251 A1 | 15 September 2016 |
| CN 108136214 A | 08 June 2018 | EP 3277381 A4 | 05 December 2018 |
| | | US 2018086693 A1 | 29 March 2018 |
| | | WO 2016161342 A2 | 06 October 2016 |
| | | WO 2016161342 A3 | 10 November 2016 |
| | | IL 254769 D0 | 31 December 2017 |
| | | AU 2016244000 A1 | 12 October 2017 |
| | | SG 11201707375U A | 30 October 2017 |
| | | CA 2981494 A1 | 06 October 2016 |
| | | TW 201706267 A | 16 February 2017 |
| | | KR 20170127463 A | 21 November 2017 |
| | | JP 2018511612 A | 26 April 2018 |
| | | BR 112017021167 A2 | 03 July 2018 |
| | | EP 3277381 A2 | 07 February 2018 |
| WO 2017087428 A1 | 26 May 2017 | BR 112017025778 A2 | 14 August 2018 |
| | | TW 201726695 A | 01 August 2017 |
| | | EP 3390415 A4 | 22 May 2019 |
| | | CA 2990696 A1 | 26 May 2017 |
| | | KR 101985778 B1 | 04 June 2019 |
| | | IL 256569 D0 | 28 February 2018 |
| | | EP 3390415 A1 | 24 October 2018 |
| | | KR 20170130615 A | 28 November 2017 |
| | | JP 2018517710 A | 05 July 2018 |
| | | AU 2016357728 A1 | 30 November 2017 |
| | | CN 108290911 A | 17 July 2018 |
| CN 103827133 A | 28 May 2014 | EA 025518 B9 | 31 October 2017 |
| | | AP 201407598 A0 | 30 April 2014 |
| | | ES 2560257 T3 | 18 February 2016 |
| | | US 9487554 B2 | 08 November 2016 |
| | | AU 2012314627 B2 | 18 August 2016 |
| | | UA 107550 C2 | 12 January 2015 |
| | | CA 2850047 C | 29 March 2016 |
| | | JP 5791810 B2 | 07 October 2015 |
| | | JO 3122 B1 | 20 September 2017 |
| | | SG 11201400144S A | 29 May 2014 |
| | | TN 2014000131 A1 | 01 July 2015 |
| | | BR 112014007223 A2 | 04 April 2017 |
| | | EP 2760878 B1 | 28 October 2015 |

Form PCT/ISA/210 (patent family annex) (January 2015)

**INTERNATIONAL SEARCH REPORT**
**Information on patent family members**

International application No.
**PCT/CN2019/084604**

| Patent document cited in search report | Publication date (day/month/year) | Patent family member(s) | Publication date (day/month/year) |
|---|---|---|---|
| | | TW I537284 B | 11 June 2016 |
| | | AP 201407598 D0 | 30 April 2014 |
| | | CN 103827133 B | 25 November 2015 |
| | | EC SP14013275 A | 30 April 2014 |
| | | GT 201400059 A | 17 February 2015 |
| | | ZA 201401568 B | 30 November 2016 |
| | | EA 201490653 A1 | 30 September 2014 |
| | | JP 2014527994 A | 23 October 2014 |
| | | HU E026645 T2 | 28 July 2016 |
| | | US 2014249119 A1 | 04 September 2014 |
| | | CL 2014000775 A1 | 18 August 2014 |
| | | KR 20140069013 A | 09 June 2014 |
| | | HK 1197414 A1 | 12 August 2016 |
| | | SI EP2760878 T1 | 29 February 2016 |
| | | NZ 622081 A | 30 October 2015 |
| | | UY 34356 A | 30 April 2013 |
| | | AR 088180 A1 | 14 May 2014 |
| | | RS 54521 B1 | 30 June 2016 |
| | | CU 20140037 A7 | 30 July 2014 |
| | | EP 2760878 A1 | 06 August 2014 |
| | | MA 35455 B1 | 01 September 2014 |
| | | PE 15542014 A1 | 24 October 2014 |
| | | EA 025518 B1 | 30 December 2016 |
| | | CA 2850047 A1 | 04 April 2013 |
| | | AU 2012314627 A1 | 10 April 2014 |
| | | CY 1117123 T1 | 05 April 2017 |
| | | IL 231755 A | 30 June 2016 |
| | | MX 2014003823 A | 04 June 2014 |
| | | DO P2014000059 A | 15 June 2014 |
| | | CO 6920294 A2 | 10 April 2014 |
| | | DE 102011083725 A1 | 04 April 2013 |
| | | WO 2013045407 A1 | 04 April 2013 |
| | | DK 2760878 T3 | 25 January 2016 |
| | | TW 201317253 A | 01 May 2013 |
| | | HR P20160050 T1 | 12 February 2016 |
| | | SI 2760878 T1 | 29 February 2016 |
| US 5424424 A | 13 June 1995 | US 5604220 A | 18 February 1997 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016021581 W
- US 2016025665 W
- US 2016062114 W
- WO 2016145092 A
- WO 2016161342 A
- WO 2017087428 A
- CN 201680015078
- CN 107530556 A
- CN 201680020013
- CN 108136214 A


### Non-patent literature cited in the description


- **SAMAD T A ; MOORE K A ; SAPIRSTEIN A et al.** Interleukin-1[beta]-mediated induction of Cox-2 in the CNS contributes to inflammatory pain hypersensitivity[J. *Nature,* 2001, vol. 410 (6827), 471-5
- **BAOJIAN Z ; YANBING Y ; GELE A et al.** Tanshinone IIA Attenuates Diabetic Peripheral Neuropathic Pain in Experimental Rats via Inhibiting Inflammation[J. *Evidence-Based Complementary and Alternative Medicine,* 2018, vol. 2018, 1-8
- **LOVERING A ; RIDE J ; BUNCE C et al.** Crystal Structures of Prostaglandin D2 11-Ketoreductase (AKR1C3) in Complex with the Nonsteroidal Anti-Inflammatory Drugs Flufenamic Acid and Indomethacin[J. *Cancer Research,* 2004, vol. 64 (5), 1802-1810
- **MATSUURA K ; SHIRAISHI H ; HARA A et al.** Identification of a principal mRNA species for human 3alpha-hydroxysteroid dehydrogenase isoform (AKR1C3) that exhibits high prostaglandin D2 11-ketoreductase activity.[J. *Journal of Biochemistry,* 1998, vol. 124 (5), 940-6
- **NOGRADY.** Medicinal Chemistry A Biochemical Approach. Oxford University Press, 1985, 388-392